# EUROPEAN PATENT APPLICATION

(11) **EP 4 079 842 A1**
(43) Date of publication of application: **26.10.2022**
(21) Application number: 20904181.3
(22) Date of filing: 16.12.2020
(51) Int. Cl.: C12N 5/0789, C07D 519/00

(54) **SMALL MOLECULE COMPOUNDS FOR AMPLIFYING HEMATOPOIETIC STEM CELLS, AND COMBINATION THEREOF**

(30) Priority: 16.12.2019 WO PCT/CN2019/125687
(71) Applicant: EdiGene (GuangZhou) Inc., Guangzhou City, Guangdong 510000 (CN)
(72) Inventor: FANG, Riguo, Beijing 102206 (CN); YANG, Huihui, Beijing 102206 (CN); SHI, Zhongyu, Beijing 102206 (CN); YUAN, Pengfei, Beijing 102206 (CN); YU, Lingling, Beijing 102206 (CN)
(74) Representative: Lavoix
(86) International application number: PCT/CN2020/136790
(87) International publication number: WO 2021/121266

(57) **Abstract**

Provided are small molecule inhibitors for amplifying hematopoietic stem cells (HSCs) and a combination thereof. The small molecule inhibitors and the combination thereof can maintain the sternness of hematopoietic stem cells while promoting the in vitro amplification of hematopoietic stem cells (HSCs).

## Description

### TECHNICAL FIELD

The present application relates to the field of biomedicine, particularly to a small molecule compound for expanding hematopoietic stem cells (HSCs), more particularly to a small molecule inhibitor of cell signaling pathway, a composition thereof, and use of the small molecule inhibitor and the composition thereof in expanding hematopoietic stem cells.

### BACKGROUND ART

Hematopoietic stem cells (HSCs) are a group of heterogeneous primitive hematopoietic cells in the blood system, with two important characteristics of self-renewal and multi-lineage differentiation. When the body is in a healthy state, HSCs in the body are in a quiescent state for a long time. When the body is in a state of disease or severe blood loss, HSCs are activated and enter a state of self-renewal and multi-lineage differentiation to maintain blood system stability and body homeostasis. The self-renewal property of HSCs is beneficial to maintain the sternness of progeny HSCs, while the multi-lineage differentiation property of HSCs allows them to differentiate into a variety of mature blood cells, such as myeloid cells (granulocytes, monocytes, erythrocytes and platelets), lymphoid cells (T cells and B cells). The properties of HSCs facilitate the differentiation of HSCs when the body needs them.

These properties of HSCs make it possible to treat hematological diseases by hematopoietic stem cell transplantation (HSCT). HSCs include long term hematopoietic stem cells (LT-HSCs) and short term hematopoietic stem cells (ST-HSCs). The former has high self-renewal ability and can carry out hematopoietic reconstruction throughout the life cycle of the body; while the latter can only maintain the function of hematopoietic reconstruction for a limited time. In 1959, Thomas et al. used bone marrow hematopoietic stem cells for the first hematopoietic stem cell transplantation in human history, clinically treating leukemia to restore normal hematopoietic function in a patient. In the following decades, through the continuous efforts of scientific researchers, hematopoietic stem cell transplantation has not only been used to treat a variety of blood system diseases, but also used to treat immunodeficiency diseases and neurodegenerative diseases.

At present, there are three main sources of HSCs, i.e., bone marrow (BM), mobilized peripheral blood (mPB), and umbilical cord blood (CB). The collection of bone marrow hematopoietic stem cells is invasive and insufficient, so this method is basically eliminated. The proportion of HSCs in human peripheral blood is very low (less than 0.1%), and it is necessary to use granulocyte colony stimulating factor (G-CSF) to mobilize hematopoietic stem cells to migrate from bone marrow to peripheral blood for transplantation. In clinical applications, the mobilization effect is often ineffective, and the number of HSCs contained in peripheral blood is insufficient, resulting in repeated mobilizations or transplantation failure. Moreover, the HSCs collected by these two methods all require human leukocyte antigen (HLA) matching between the donor and the patient. HLA matching is difficult, and once mismatch occurs, it will lead to graft-versus-host reaction (GVHD), and a patient with GVHD will die from immune system disorders.

As a new source of HSCs, umbilical cord blood has many advantages. Firstly, umbilical cord blood HSCs have low requirements for HLA matching, allowing partial HLA mismatch, and the incidence of GVHD after transplantation is low, thereby alleviating the difficulty of traditional HSCT matching; secondly, umbilical cord blood collection is convenient, harmless to the donor and has no ethical issues, and HSCs have strong hematopoietic ability. These advantages make umbilical cord blood a preferred source of HSCs for treating diseases in future.

However, due to the small amount of cells in a single collection of umbilical cord blood, the overall number of HSCs is small, so it is limited to children or light-weight adults for HSCs transplantation, and cannot meet the transplant needs of larger-weight adults. When the number of transplanted HSCs is insufficient, neutrophil recovery is delayed in a patient, leading to an increased risk of GVHD. These have become bottlenecks restricting the clinical application of umbilical cord blood.

In conclusion, the safety and efficacy of hematopoietic stem cell transplantation depend on the content of transplanted HSCs. If the number of HSCs can be expanded in vitro, the success rate of hematopoietic stem cell transplantation can be improved.

Researchers continue to work hard and try to explore different methods to achieve in vitro expansion of hematopoietic stem cells. One of the strategies for in vitro expansion of HSCs is to utilize small-molecule compounds (SMCs) targeting HSCs. SMCs are readily available in source, easy to mass produce, stable in nature, clear in structure, and easy to control in concentration, and have been widely used in medical research. In the current HSCs in vitro expansion technology, SMCs can significantly increase the expansion folds of HSCs. For example, the AhR aryl hydrocarbon receptor inhibitor StemRegenin1 (SRI) is the first screened SMC that can expand HSCs in vitro. The pyrimidine indole derivative UM171 is also able to expand HSCs in vitro, but it does not act through the AhR cell signaling pathway. Transcriptome analysis shows that, UM171 does not down-regulate AhR cell signaling pathway, but inhibits erythrocyte and megakaryocyte differentiation-related genes. The combination of the two can increase the amplification folds of HSCs.

Histone deacetylases (HDACs) are known cell signaling pathways. Histones can regulate the transcriptional process of specific genes, cell proliferation and differentiation through acetylation or deacetylation. Existing research results reveal that, histone acetylation plays a role in the self-renewal and proliferation of HSCs. HDAC inhibitors TSA, trapoxin and chlamydocin, can regulate histone acetylation in vitro to promote the self-renewal and proliferation of HSCs.

Src is encoded by the Src proto-oncogene, and is a non-receptor protein kinase with tyrosine protein kinase activity. It exists in the cytoplasm, and can be activated by a variety of cell surface receptors to participate in mediating multiple cell signaling pathways, thereby regulating cell proliferation, differentiation and other processes, and it is a key molecule in multiple cell signaling pathways. For example, after Src is activated, it cooperates with p52Shc to activate the mitogen-activated protein kinase (MAPK) cell signaling pathway, and participates in the downstream of MAPK to regulate cell growth and differentiation process; Src can also activate the STAT cell signaling pathway and promote the transcription of related genes. So far, no studies have reported that Src inhibitors help to maintain the sternness of HSCs during in vitro expansion, but in the study of the present invention we found that Src inhibitors can promote the proliferation of HSCs and maintain the sternness of HSCs.

Although it has been found that small molecule inhibitors of some cell signaling pathways can promote the proliferation of HSCs, in this field it still needs to continue to search for small molecule compounds that can promote the proliferation of HSCs more effectively and can maintain the sternness of the proliferated HSCs, and a comprehensive strategy to achieve this purpose, so as to meet the clinical needs.

### SUMMARY OF THE INVENTION

The problem to be solved by this application is to screen out the best small molecule compounds and their compositions that can promote the in vitro expansion of HSCs while maintaining a high proportion of sternness of HSCs by studying the key factors that regulate cell signaling pathways, so as to solve the problem in the prior art that the number of HSCs expanded in vitro is still insufficient.

Firstly, the applicant's research found that multiple small-molecule inhibitors targeting Src in the STAT cell signaling pathway can well maintain the sternness of HSCs in vitro culture. Src targets play an important role in the expansion of hematopoietic stem cells in the STAT cell signaling pathway, while maintaining the sternness of hematopoietic stem cells, which has not been reported in previous studies.

Further, the applicant found that during the in vitro culture of HSCs, the combinations of HDAC inhibitors, such as SAHA and Valproic acid (VPA), with small molecule inhibitors targeting Src can well maintain the sternness of HSCs, and its effect is far better than SRI and UM171 which have been found in the prior art.

Therefore, in one aspect the present application finds that small molecule inhibitors of the cell signaling pathway of signal transducer and activator of transcription (STAT), such as a small molecule inhibitor targeting Src target, can promote the proliferation of HSCs and maintain the sternness characteristics of HSCs. In some embodiments, the present application provides a method for promoting the proliferation of HSCs and maintaining the sternness of HSCs, comprising: in vitro contacting the HSCs with a culture medium containing a small molecule inhibitor of the STAT cell signaling pathway or a small molecule inhibitor of other cell signaling pathway than the STAT cell signaling pathway. In some embodiments, the small molecule inhibitor of the STAT cell signaling pathway is a small molecule inhibitor targeting Src. In some embodiments, the small molecule inhibitor targeting Src is one or more selected from the group consisting of: Dasatinib, Quercetin, UM-164, KX2-391, and KX1-004. In some embodiments, the small molecule inhibitor targeting Src is one or more selected from the group consisting of: Dasatinib, UM-164, and KX1-004.

In some embodiments, the small molecule inhibitor targeting Src is used in combination with the small molecule inhibitor of the other cell signaling pathway. In some embodiments, the small molecule inhibitor of the other cell signaling pathway is one or two or more selected from the group consisting of: a small molecule inhibitor targeting HDAC, a small molecule inhibitor targeting PKC, and a small molecule inhibitor targeting JNK. In some embodiments, the small molecule inhibitor targeting Src is used in combination with a small molecule inhibitor targeting HDAC, a small molecule inhibitor targeting PKC, or a small molecule inhibitor targeting JNK. In some embodiments, the small molecule inhibitor targeting Src is used in combination with a small molecule inhibitor VPA targeting HDAC, a small molecule inhibitor SAHA targeting HDAC, a small molecule inhibitor Enzastaurin targeting PKC, or a small molecule inhibitor JNK-IN-8 targeting JNK. In some embodiments, the small molecule inhibitor Dasatinib targeting Src is used in combination with VPA, or SAHA. In some embodiments, the small molecule inhibitor of the cell signaling pathway of signal transducer and activator of transcription (STAT), e.g., a small molecule inhibitor targeting Src, such as Dasatinib, UM-164 or KX1-004, maintains the HSCs with a CD34+CD45+CD90+CD45RA-CD38- phenotype accounting for more than 8%, 10%, 15%, 20%, 25%, or 30% of all cells; for example, maintains HSCs with a CD34+CD45+CD90+CD45RA-CD38- phenotype accounting for 5%-35%, 10%-35%, 15%-35%, 20%-35%, 25%-35%, 5%-30%, 10%-30%, 20%-30%, 5%-25%, 10%-25%, 15%-25%, 20%-25%, 5%-20%, 10%-20%, 15%-20% of all cells; maintains CD34+ cells accounting for more than 65%, 70%, 75%, 80%, or 85% of all cells.

In some embodiments, the present application provides a composition for maintaining the sternness of HSCs, comprising a small molecule inhibitor of the STAT cell signaling pathway. In some embodiments, the small molecule inhibitor of the STAT cell signaling pathway is a small molecule inhibitor targeting Src. In some embodiments, the small molecule inhibitor targeting Src is one or more selected from the group consisting of: Dasatinib, Quercetin, UM-164, KX2-391, and KX1-004. In some embodiments, the composition further comprises the small molecule inhibitor of the other cell signaling pathway. In some embodiments, the small molecule inhibitor of the other cell signaling pathways comprises a small molecule inhibitor targeting HDAC, a small molecule inhibitor targeting PKC, or a small molecule inhibitor targeting JNK. In some embodiments, the small molecule inhibitor of the other cell signaling pathway comprises a small molecule inhibitor VPA targeting HDAC, a small molecule inhibitor SAHA targeting HDAC, a small molecule inhibitor Enzastaurin targeting PKC, or a small molecule inhibitor JNK-IN-8 targeting JNK. In some embodiments, the composition further comprises SFEMII medium, growth factor Flt-3L, growth factor SCF, growth factor TPO, and growth factor IL-6. In some embodiments, the composition consists of a small molecule inhibitor of STAT cell signaling pathway and/or a small molecule inhibitor of the other cell signaling pathway, SFEMII medium, growth factor Flt-3L, growth factor SCF, growth factor TPO, and growth factor IL-6. In some embodiments, the composition maintains HSCs with a CD34+CD45+CD90+CD45RA-CD38- phenotype accounting for more than 8%, 10%, 15%, 20%, 25%, or 30% of all cells, for example, maintains HSCs with CD34+CD45+CD90+CD45RA-CD38- phenotype accounting for 5%-35%, 10%-35%, 15%-35%, 20%-35%, 25%-35%, 5%-30%, 10%-30%, 20%-30%, 5%-25%, 10%-25%, 15%-25%, 20%-25%, 5%-20%, 10%-20%, or 15%-20% of all cells. In some embodiments, the composition maintains CD34+ cells accounting for more than 65%, 70%, 75%, 80%, or 85% of all cells.

In some embodiments, the small molecule inhibitor of the cell signaling pathway of signal transducer and activator of transcription (STAT), e.g., a small molecule inhibitor targeting Src, such as Dasatinib, UM-164, or KX1-004, is used in combination with the small molecule inhibitor of the other cell signaling pathway, such as VPA or SAHA, to maintain HSCs with a CD34+CD45+CD90+CD45RA-CD38- phenotype accounting for more than 8%, 10%, 15%, 20%, 25%, or 30% of all cells; for example, to maintain HSCs with a CD34+CD45+CD90+CD45RA-CD38- phenotype accounting for 5%-35%, 10%-35%, 15%-35%, 20%-35%, 25%-35%, 5%-30%, 10%-30%, 20%-30%, 5%-25%, 10%-25%, 15%-25%, 20%-25%, 5%-20%, 10%-20%, or 15%-20% of all cells; to maintain CD34+ cells accounting for more than 65%, 70%, 75%, 80%, or 85% of all cells. In some embodiments, the concentration of each inhibitor in the culture medium is:
Dasatinib: 0.1µM-50µM, preferably 0.5µM-40µM, more preferably 0.5µM-30µM, most preferably 0.5µM-10µM;
SAHA: 10nM-20µM, preferably 20nM-15µM, more preferably 30nM-10µM, most preferably 01µM-10µM;
VPA: 10µM-2000µM, preferably 10µM-1500µM, more preferably 10µM-1000µM, most preferably 100µM-1000µM;
JNK-IN-8: 0.1µM-20µM, preferably 0.5µM-15µM, more preferably 0.5µM-10µM, most preferably 1µM-10µM;
EPZ004777: 0.1µM-50µM, preferably 0.5µM-40µM, more preferably 0.5µM-30µM, most preferably 0.5µM-10µM;
DZNeP: 1nM-500nM, preferably 5nM-400nM, more preferably 10nM-300nM, most preferably 10nM-250nM;
UM-164: 0.1µM-1000µM, preferably 0.5µM-500µM, more preferably 1µM-100µM, most preferably 1µM-10µM;
KX2-391: 0.1nM-1000nM, preferably 1nM-1000nM, more preferably 10nM-500nM, most preferably 10nM-100nM;
KX1-004: 0.1µM-1000µM, preferably 1µM-1000µM, more preferably 10µM-500µM, most preferably 10µM-100µM.

In one aspect, the present application provides a method for promoting the proliferation of HSCs and maintaining the sternness of the HSCs, comprising: in vitro contacting the HSCs with a culture medium containing one or more small molecule inhibitors selected from the group consisting of: 1) a small molecule inhibitor VPA targeting HDAC; 2) a small molecule inhibitor SAHA targeting HDAC; 3) a small molecule inhibitor Enzastaurin targeting PKC; and 4) a small molecule inhibitor JNK-IN-8 targeting JNK.

In one aspect, the present application provides a composition for maintaining the sternness of HSCs, wherein the composition comprises any combination selected from the group consisting of: SAHA+EPZ004777, SAHA+DZNeP, SAHA+Dasatinib, VPA+Dasatinib, SAHA+JNK-IN-8, or SAHA+VPA. In some embodiments, the composition further comprises SFEMII medium, growth factor Flt-3L, growth factor SCF, growth factor TPO, and growth factor IL-6. In some embodiments, the composition consists of: any combination selected from SAHA+EPZ004777, SAHA+DZNeP, SAHA+Dasatinib, VPA+Dasatinib, SAHA+JNK-IN-8 or SAHA+VPA, and SFEMII medium, growth Factor Flt-3L, growth factor SCF, growth factor TPO, and growth factor IL-6. In some embodiments, the composition for maintaining the sternness of HSCs may also facilitate to maintain the proportion of CD34+ cells. In some embodiments, the composition maintains HSCs with a CD34+CD45+CD90+CD45RA-CD38- phenotype accounting for more than 8%, 10%, 15%, 20%, 25%, or 30% of all cells, for example, maintains HSCs with CD34+CD45+CD90+CD45RA-CD38-phenotype accounting for 5%-35%, 10%-35%, 15%-35%, 20%-35%, 25%-35%, 5%-30%, 10%-30%, 20%-30%, 5%-25%, 10%-25%, 15%-25%, 20%-25%, 5%-20%, 10%-20%, or 15%-20% of all cells; maintains CD34+ cells accounting for more than 65%, 70%, 75%, 80%, or 85% of all cells. In some embodiments, the concentration of each inhibitor in the culture medium is:
Dasatinib: 0.1µM-50µM, preferably 0.5µM-40µM, more preferably 0.5µM-30µM, most preferably 0.5µM-10µM;
SAHA: 10nM-20µM, preferably 20nM-15µM, more preferably 30nM-10µM, most preferably 0.1µm-10µM;
VPA: 10µM-2000µM, preferably 10µM-1500µM, more preferably 10µM-1000µM, most preferably 100µM-1000µM;
JNK-IN-8: 0.1µM-20µM, preferably 0.5µM-15µM, more preferably 0.5µM-10µM, most preferably 1 µM-10µM;
EPZ004777: 0.1µM-50µM, preferably 0.5µM-40µM, more preferably 0.5µM-30µM, most preferably 0.5µM-10µM;
DZNeP: 1nM-500nM, preferably 5nM-400nM, more preferably 10nM-300nM, most preferably 10nM-250nM.

In one aspect, the present application provides a composition for maintaining the sternness of HSCs, wherein the composition comprises any combination selected from: SAHA+EPZ004777+DZNeP, or SAHA+VPA+Dasatinib. In some embodiments, the composition further comprises SFEMII medium, growth factor Flt-3L, growth factor SCF, growth factor TPO, and growth factor IL-6. In some embodiments, the composition consists of: SAHA+EPZ004777+DZNeP or SAHA+VPA+Dasatinib, and SFEMII medium, growth Factor Flt-3L, growth factor SCF, growth factor TPO, and growth factor IL-6. In some embodiments, the composition maintains HSCs with a CD34+CD45+CD90+CD45RA-CD38- phenotype accounting for more than 8%, 10%, 15%, 20%, 25%, or 30% of all cells, for example, maintains HSCs with CD34+CD45+CD90+CD45RA-CD38- phenotype accounting for 5%-35%, 10%-35%, 15%-35%, 20%-35%, 25%-35%, 5%-30%, 10%-30%, 20%-30%, 5%-25%, 10%-25%, 15%-25%, 20%-25%, 5%-20%, 10%-20%, or 15%-20% of all cells; maintains CD34+ cells accounting for more than 65%, 70%, 75%, 80%, or 85% of all cells. In some embodiments, the concentration of each inhibitor in the culture medium is:
Dasatinib: 0.1µM-50µM, preferably 0.5µM-40µM, more preferably 0.5µM-30µM, most preferably 0.5µM-10µM;
SAHA: 10nM-20µM, preferably 20nM-15µM, more preferably 30nm-10µM, most preferably 0.1µm-10µM;
VPA: 10µM-2000µM, preferably 10µM-1500µM, more preferably 10µM-1000µM, most preferably 100µM-1000µM;
EPZ004777: 0.1µM-50µM, preferably 0.5µM-40µM, more preferably 0.5µM-30µM, most preferably 0.5µM-10µM;
DZNeP: 1nM-500nM, preferably 5nM-400nM, more preferably 10nM-300nM, most preferably 10nM-250nM.

In some embodiments, the small molecule inhibitor of the STAT cell signaling pathway is a small molecule inhibitor targeting Src. In some embodiments, the small molecule inhibitor targeting Src is one or more selected from the group consisting of: Dasatinib, Quercetin, UM-164, KX2-391, and KX1-004. In some embodiments, the small molecule inhibitor targeting Src is one or more selected from the group consisting of: Dasatinib, UM-164, and KX1-004. In the applicant's research results, the above small molecule inhibitors of cell signaling pathways can well maintain the sternness of HSCs during in vitro expansion and the proportion of CD34+ cells, and the effect of the combinations of these small molecule inhibitors is superior to the small molecule inhibitor combinations reported in the prior art in the self-renewal and sternness maintenance of HSCs.

In this application, the applicant has found that a Src inhibitor can well maintain the sternness of HSCs in the in vitro expansion and culture of HSCs; and it was found that the effect of the combination of an inhibitor targeting HDAC and an inhibitor targeting Src is better than the effect of the combination of the small molecule inhibitors reported in the prior art and the effect of these small molecule inhibitors used alone in the in vitro expansion and culture of HSCs. The applicant's research results can realize the in vitro expansion of HSCs while maintaining a high proportion of sternness of HSCs, laying a foundation for the clinical application of HSCs.

The above-mentioned "sternness" of hematopoietic stem cells is an abbreviation for the characteristics of hematopoietic stem cells. Hematopoietic stem cells (HSCs) exhibit two major cell biological characteristics: self-renewal capacity and pluripotency. These properties of hematopoietic stem cells are referred to as "sternness" for short. To some extent, molecular phenotypes expressed on the cell surface of hematopoietic stem cells can reflect whether they maintain "sternness". For example, if the phenotype of hematopoietic stem cells is CD34+CD45+CD90+CD45RA-CD38-, it means that they are LT-HSCs and maintain "sternness". In this application, hematopoietic stem cells with CD34+CD45+CD90+CD45RA-CD38- phenotype are defined as LT-HSCs; if hematopoietic stem cells have CD34+CD45+CD90+CD45RA-CD38- phenotype, they are defined as maintaining or retaining the properties of hematopoietic stem cells, i.e., "sternness".

The total cells refer to all progeny cells after culturing the initial CD34+ cells.

In the present invention, hematopoietic stem cells are contacted in vitro with a small molecule inhibitor of STAT cell signal transduction pathway, such as a small molecule inhibitor targeting Src or the small molecule inhibitor of the other cell signaling pathway, such as a small molecule inhibitor targeting HDAC, a small molecule inhibitor targeting PKC, and a small molecule inhibitor targeting JNK, thereby well maintaining the sternness of HSCs during in vitro expansion and the proportion of CD34+ cells in all HSCs, and in self-renewal, sternness maintenance and other aspects of HSCs, the effect of combinations of these small molecule inhibitors is superior to the effect of the small molecule combinations reported in the prior art, and the transplantation efficiency of cells treated with the above-mentioned small molecule inhibitors is significantly higher than the transplantation efficiency of cells treated with small molecule inhibitors reported in the prior art.

### BRIEF DESCRIPTION OF THE DRAWINGS

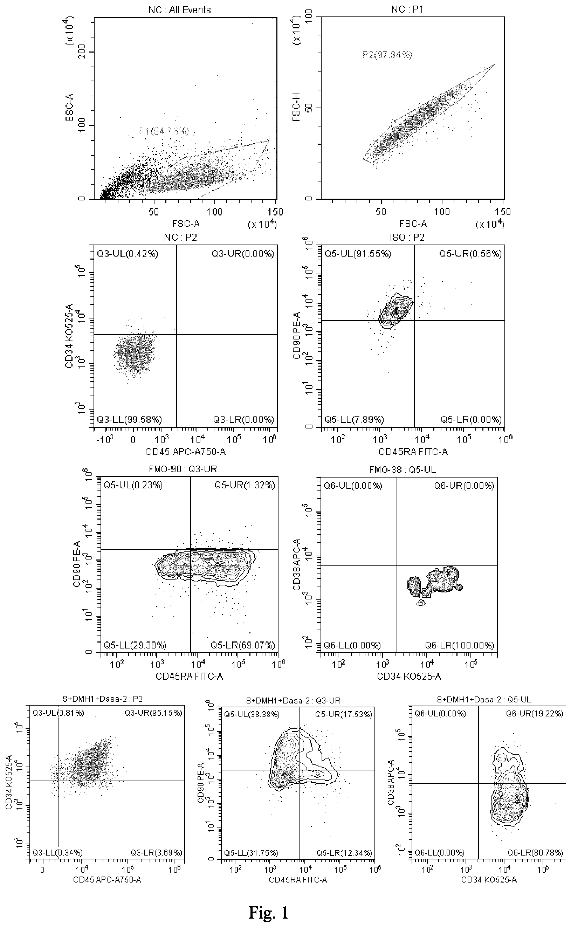

Fig. 1 shows the determination of the logic gate and gate position of the target cell population, i.e., CD34+CD45+CD45RA-CD90+CD38- cell population.

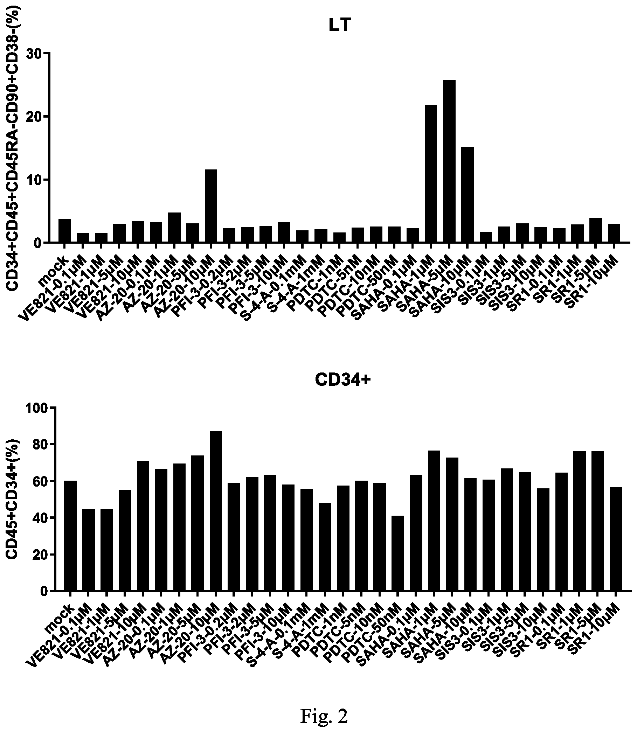

Fig. 2 shows the first round screening for optimal concentrations of small molecule inhibitors and for the ability to maintain the sternness of HSCs on umbilical cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitors in Table 4 (4 concentrations are tested for each small molecule inhibitor), wherein the abscissa represents the name of the inhibitor and the corresponding concentration, and the ordinate CD34+CD45+CD90 +CD45RA-CD38-(%) represents the proportion of LT-HSCs in all cells, and CD34+CD45+(%) represents the purity of HSCs.

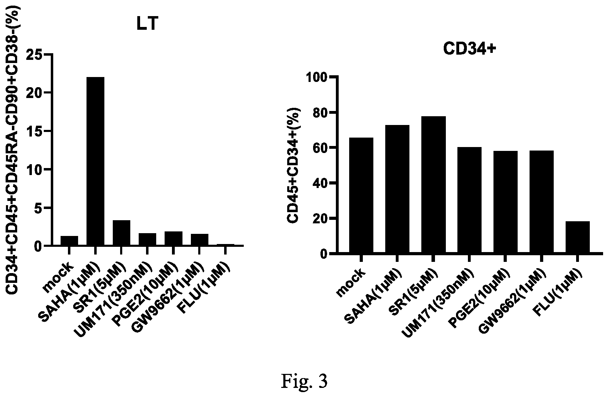

Fig. 3 shows the second round screening for optimal concentrations of small molecule inhibitors and for the ability to maintain the sternness of HSCs on umbilical cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitors in Table 5, wherein the abscissa represents the names of the inhibitor and the corresponding concentration, and the ordinate CD34+CD45+CD90+CD45RA-CD38-(%) represents the proportion of LT-HSCs in all cells, CD34+CD45+ (%) represents the purity of HSCs.

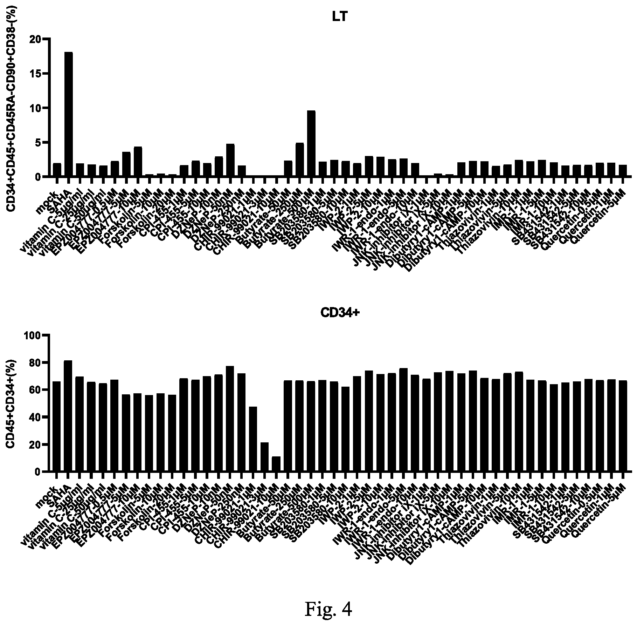

Fig. 4 shows the third round screening for optimal concentrations of small molecule inhibitors and for the ability to maintain the sternness of HSCs on umbilical cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitors in Table 6 (except for SAHA-1µM, 3 concentrations are tested for each inhibitor), wherein the abscissa represents the name of the inhibitor and the corresponding concentration, and the ordinate CD34+CD45+CD90+CD45RA-CD38-(%) represents the proportion of LT-HSCs in all cells, CD34+CD45+ (%) represents the purity of HSCs.

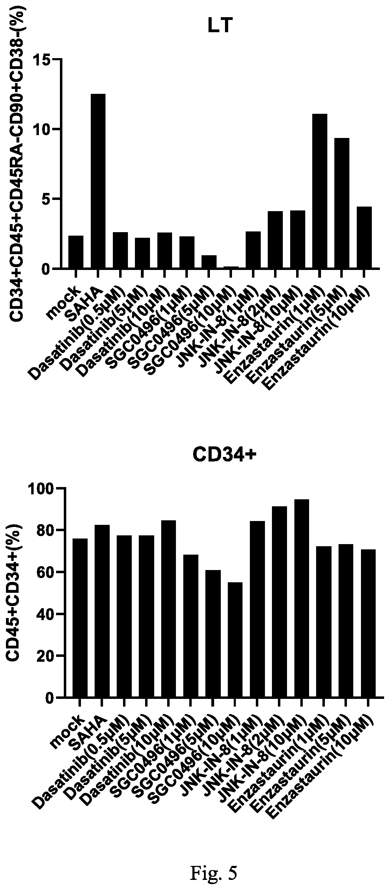

Fig. 5 shows the fourth round screening for optimal concentrations of small molecule inhibitors and for the ability to maintain the sternness of HSCs on umbilical cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitors in Table 7 (except for SAHA-1µM, 3 concentrations are tested for each inhibitor), wherein the abscissa represents the name of the inhibitor and the corresponding concentration, and the ordinate CD34+CD45+CD90+CD45RA-CD38-(%) represents the proportion of LT-HSCs in all cells, CD34+CD45+ (%) represents the purity of HSCs.

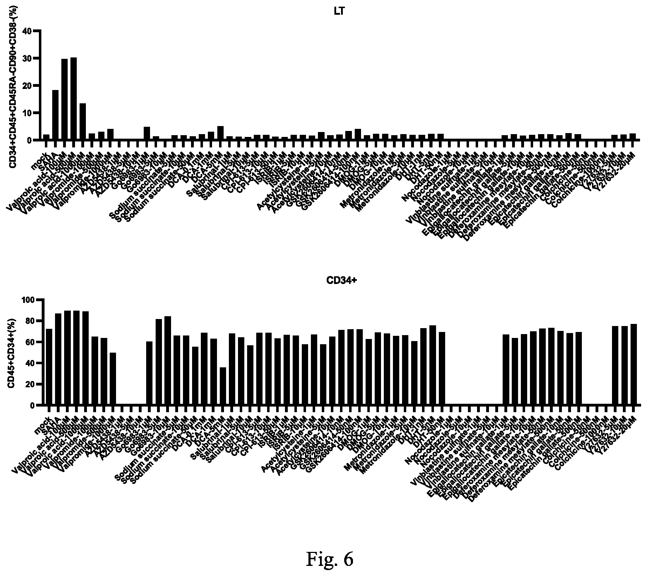

Fig. 6 shows the fifth round screening for optimal concentrations of small molecule inhibitors and for the ability to maintain the sternness of HSCs on umbilical cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitors in Table 8 (except for SAHA-1 µM, 3 concentrations are tested for each inhibitor), wherein the abscissa represents the name of the inhibitor and the corresponding concentration, and the ordinate CD34+CD45+CD90+CD45RA-CD38-(%) represents the proportion of LT-HSCs in all cells, CD34+CD45+ (%) represents the purity of HSCs.

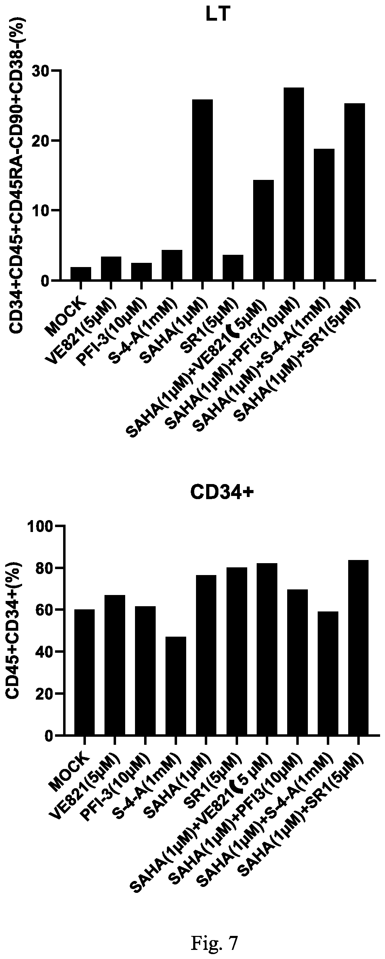

Fig. 7 shows the first round of screening for the best bimolecular combination of small molecule inhibitors to maintain sternness of HSCs on cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitor combinations: SAHA+SR1, SAHA+VE821, SAHA+PFI3 and SAHA+S-4-A, wherein the abscissa represents the name of the inhibitor and the corresponding concentration, and the ordinate CD34+CD45+CD90+CD45RA-CD38-(%) represents the proportion of LT-HSCs in all cells, CD34+CD45+ (%) represents the purity of HSCs.

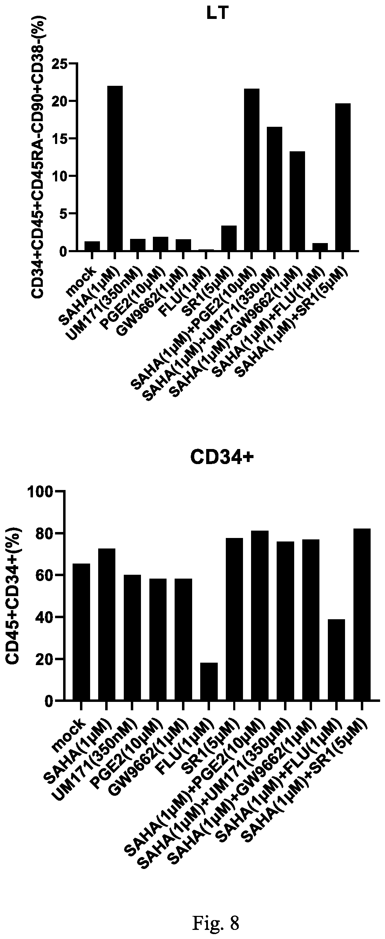

Fig. 8 shows the second round of screening for the best bimolecular combination of small molecule inhibitors to maintain sternness of HSCs on cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitor combinations: SAHA+SR1, SAHA+UM171, SAHA+PGE2, SAHA+GW9662 and SAHA+FLU, wherein the abscissa represents the name of the inhibitor and the corresponding concentration, and the ordinate CD34+CD45+CD90+CD45RA-CD38-(%) represents the proportion of LT-HSCs in all cells, CD34+CD45+ (%) represents the purity of HSCs.

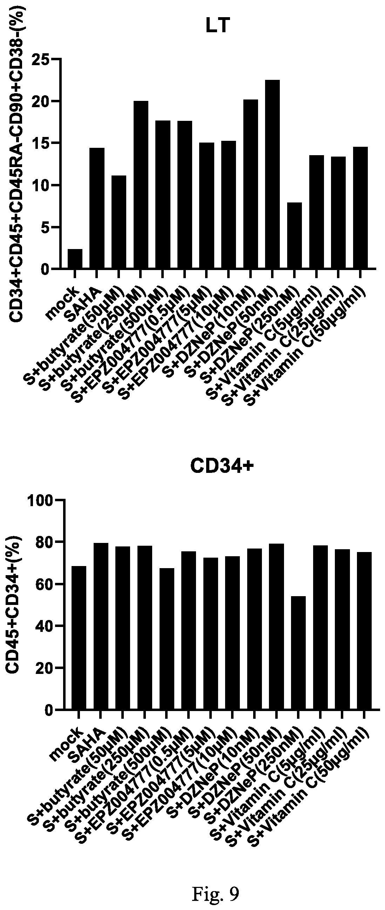

Fig. 9 shows the third round of screening for the best bimolecular combination of small molecule inhibitors to maintain sternness of HSCs on cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitor combinations: SAHA+Butyrate, SAHA+EPZ004777, SAHA+DZNeP and SAHA+Vitamin C (except for SAHA, 3 concentrations are tested for each inhibitor), wherein the abscissa represents the name of the inhibitor and the corresponding concentration, and the ordinate CD34+CD45+CD90+CD45RA-CD38-(%) represents the proportion of LT-HSCs in all cells, CD34+CD45+ (%) represents the purity of HSCs. S stands for SAHA (1 µM).

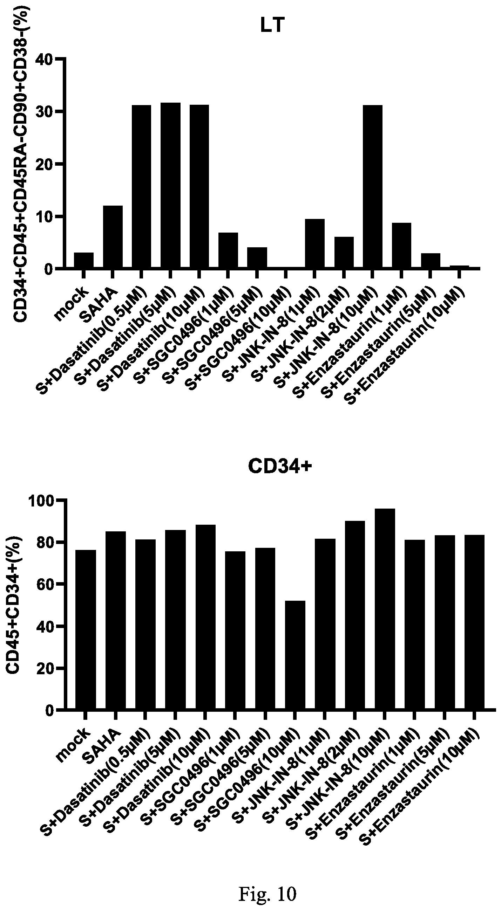

Fig. 10 shows the fourth round of screening for the best bimolecular combination of small molecule inhibitors to maintain sternness of HSCs on cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitor combinations: SAHA+Dasatinib, SAHA+SGC0496, SAHA+JNK-IN-8 and SAHA+Enzastaurin(LY317615) (except for SAHA, 3 concentrations are tested for each inhibitor), wherein the abscissa represents the name of the inhibitor and the corresponding concentration, and the ordinate CD34+CD45+CD90+CD45RA-CD38-(%) represents the proportion of LT-HSCs in all cells, CD34+CD45+ (%) represents the purity of HSCs. S stands for SAHA (1 µM).

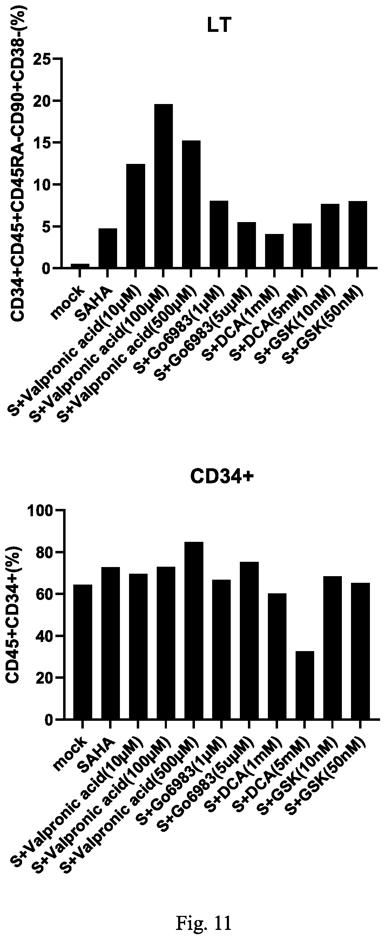

Fig. 11 shows the fifth round of screening for the best bimolecular combination of small molecule inhibitors to maintain sternness of HSCs on cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitor combinations: SAHA+VPA, SAHA+Go6983, SAHA+DCA and SAHA+GSK2606414 (except for SAHA, 2-3 concentrations are tested for each inhibitor), wherein the abscissa represents the name of the inhibitor and the corresponding concentration, and the ordinate CD34+CD45+CD90+CD45RA-CD38-(%) represents the proportion of LT-HSCs in all cells, CD34+CD45+ (%) represents the purity of HSCs. S stands for SAHA (1 µM).

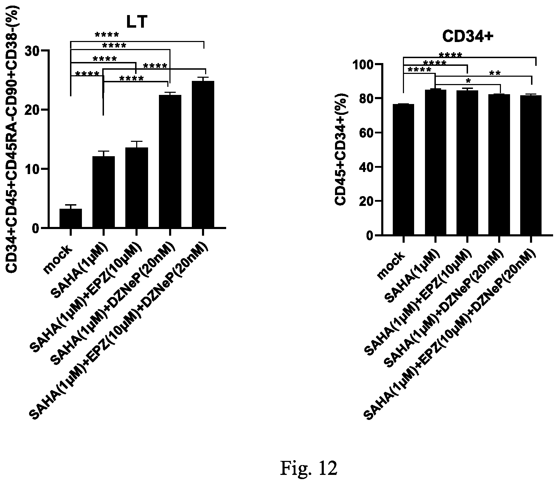

Fig. 12 shows the first round of screening for the best trimolecular combination of small molecule inhibitors to maintain sternness of HSCs on cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitor combination SAHA+EPZ004777+DZNeP, wherein the abscissa represents the names of inhibitors and the corresponding concentrations, and the ordinate CD34+CD45+CD90+CD45RA-CD38-(%) represents the proportion of LT-HSCs in all cells, CD34+CD45+ (%) represents the purity of HSCs. 3 replicates in each group, ^{∗} means significant difference.

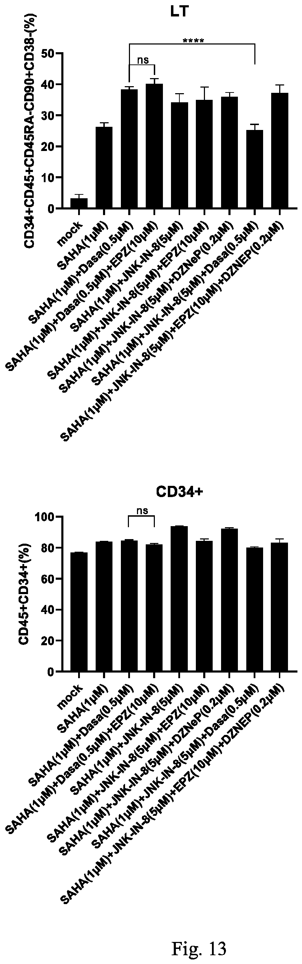

Fig. 13 shows the second round of screening for the best trimolecular combination of small molecule inhibitors to maintain sternness of HSCs on cord blood-derived CD34+ cells, the analysis diagram of expression of LT-HSCs cell surface markers (CD34+CD45+CD90+CD45RA-CD38-) detected by flow cytometry after 6-7 days of induction with small molecule combinations: SAHA+Dasatinib+EPZ004777, SAHA+JNK-IN-8+EPZ004777, SAHA+JNK-IN-8+DZNeP, SAHA+JNK-IN-8+Dasatinib and SAHA+JNK-IN-8+EPZ004777+DZNeP, wherein the abscissa represents the names of the inhibitor combinations and the corresponding concentrations, and the ordinate CD34+CD45+CD90+CD45RA-CD38-(%) represents the proportion of LT-HSCs in all cells, CD34+CD45+ (%) represents the purity of HSCs. 3 replicates in each group, ^{∗} means significant difference.

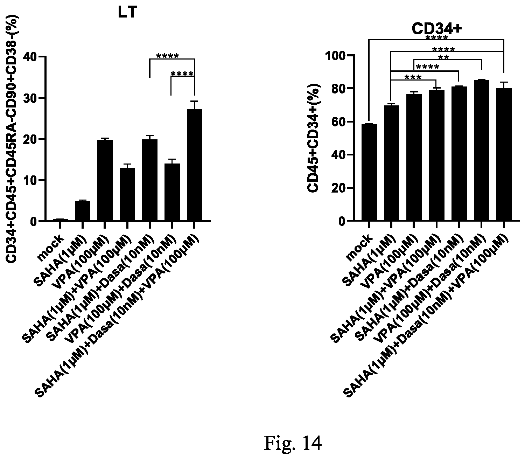

Fig. 14 shows the third round of screening for the best trimolecular combination of small molecule inhibitors to maintain sternness of HSCs on cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitor combination SAHA+VPA+Dasatinib, wherein the abscissa represents the names of the inhibitor combinations and the corresponding concentrations, and the ordinate CD34+CD45+CD90+CD45RA-CD38-(%) represents the proportion of LT-HSCs in all cells, CD34+CD45+ (%) represents the purity of HSCs. 3 replicates in each group, ^{∗} means significant difference.

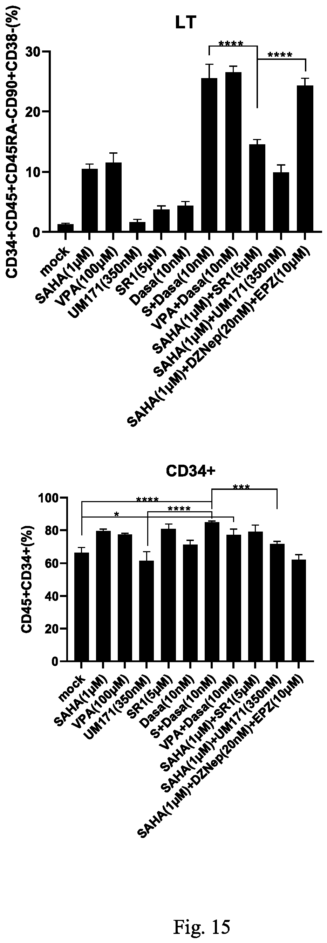

Fig. 15 shows a comparison between the screened small molecule inhibitors and the small molecule inhibitors SRI and UM171 reported in literatures on cord blood-derived CD34+ cells. The analysis diagram of expression of LT-HSCs cell surface markers (CD34+CD45+CD90+CD45RA-CD38-) detected by flow cytometry after 6-7 days of induction with small molecule inhibitors, wherein the abscissa represents the names of the inhibitor combinations and the corresponding concentrations, and the ordinate CD34+CD45+CD90+CD45RA-CD38-(%) represents the proportion of LT-HSCs in all cells, CD34+CD45+ (%) represents the purity of HSCs. 3 replicates in each group, ^{∗} means significant difference.

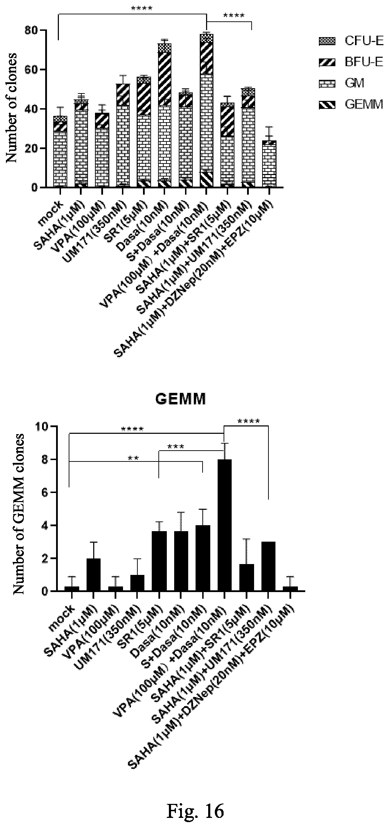

Fig. 16 shows the analysis diagram of the in vitro clonogenic ability of the screened small molecule inhibitors and the small molecule inhibitors SRI and UM171 reported in literatures on umbilical cord blood-derived CD34+ cells. BFU-E, CFU-E, CFU-GM, CFU-GEMM represent clones of different blood lineages such as erythroid, myeloid, and lymphoid, wherein the abscissa represents the names of the inhibitor combinations and the corresponding concentrations, the ordinate (colonies number) represents the total number of clones, and the colonies number of GEMM represents the number of CFU-GEMM clones. 3 replicates in each group, ^{∗} means significant difference. S stands for SAHA (1 µM).

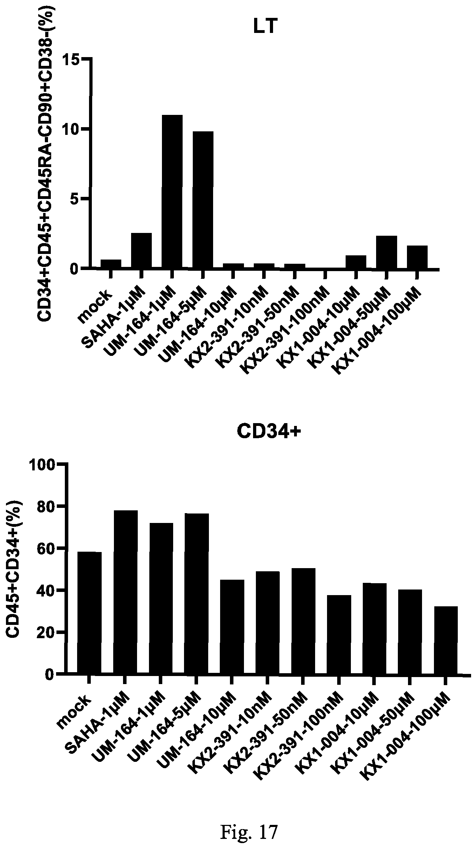

Fig. 17 shows the screening for optimal concentrations of small molecule inhibitors and for the ability to maintain the sternness of HSCs on cord blood-derived CD34+ cells, the analysis diagram of expression of LT-HSCs cell surface markers (CD34+CD45+CD90+CD45RA-CD38-) detected by flow cytometry after 6-7 days of induction with small molecule inhibitors in Table 9 (except for SAHA-1µM, 3 concentrations are tested for each inhibitor), wherein the abscissa represents the name of the inhibitor and the corresponding concentration, and the ordinate CD34+CD45+CD90+CD45RA-CD38-(%) represents the proportion of LT-HSCs in all cells, CD34+CD45+ (%) represents the purity of HSCs.

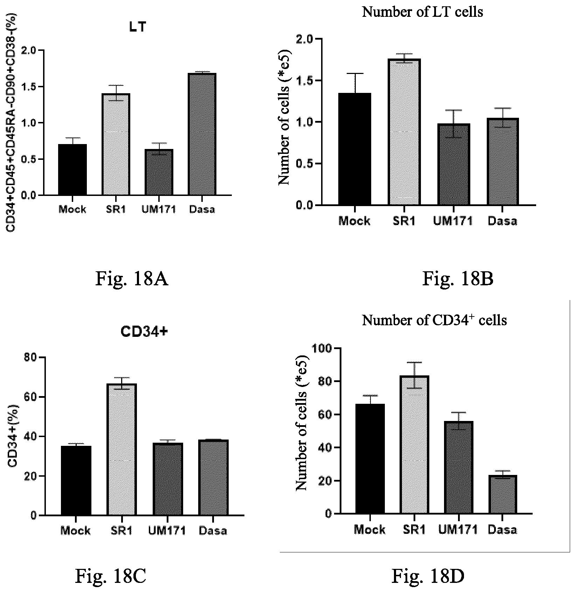

In Fig. 18, Figs. 18A and 18C show the analysis diagram of expression of LT-HSCs cell surface markers (CD34+CD45+CD90+CD45RA-CD38-) detected by flow cytometry after 8 days of treatment with small molecule: Mock (DMSO), SRI (5 µM), UM171 (350 nM) and Dasatinib (50 nM) on cord blood-derived CD34+ cells, wherein the abscissa represents the name of the inhibitor, and the ordinate CD34+CD45+CD90+CD45RA-CD38-(%) represents the proportion of LT-HSCs in all cells, CD34+CD45+(%) represents the purity of HSCs. Figs. 18B and 18D show the absolute number of LT-HSCs and CD34+ cell proliferation after 8 days of treatment with small molecule: Mock (DMSO), SRI (5 µM), UM171 (350 nM) and Dasatinib (50 nM) on cord blood-derived CD34+ cells, wherein the abscissa represents the name of the inhibitor, and the ordinate represents the number of cells.

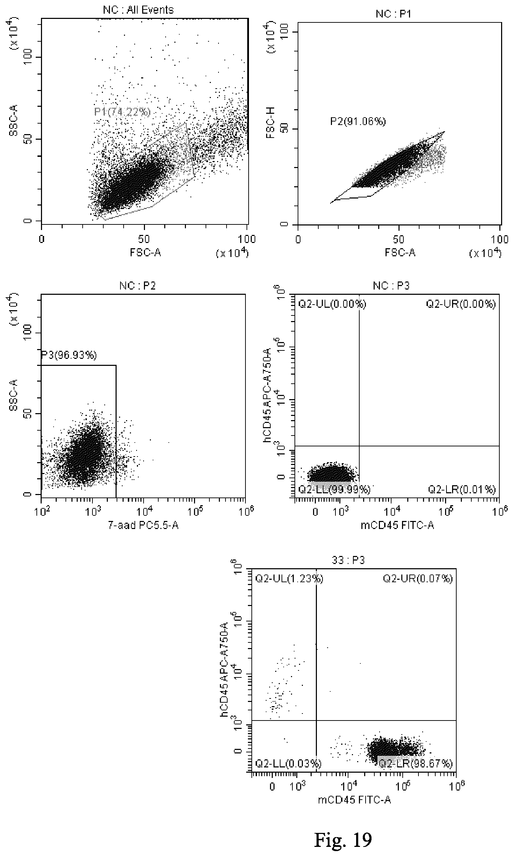

Fig. 19 shows the determination of logic gates and gate positions for hCD45+ and mCD45+ cell populations; wherein NC represents the control without adding antibody, 33 represents sample No. 33 with added antibody, hCD45 represents human-derived CD45+ cells, and mCD45 represents murine-derived CD45+ cells.

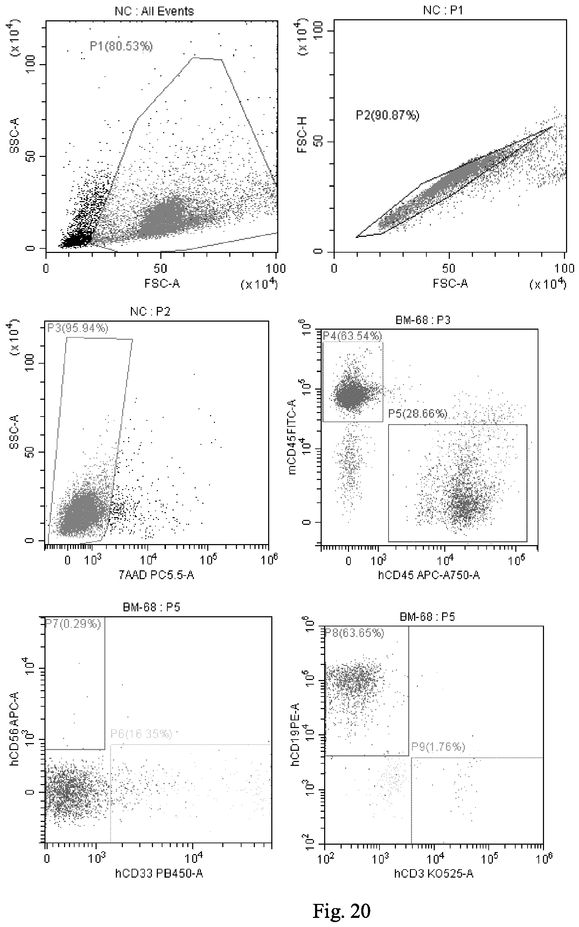

Fig. 20 shows the determination of logic gates and gate positions for hCD45+, hCD3+, hCD33+, hCD56+, hCD19+ and mCD45+ cell populations; wherein hCD3+ represents human-derived CD3+ cells and is a surface marker of T lymphocytes; hCD33+ represents human-derived CD33+ cells and is a surface marker of myeloid cells; hCD56+ represents human-derived CD56+ cells and is a surface marker of natural killer cells (NK cells); hCD19+ represents human-derived CD19+ cells and is a surface marker of B lymphocytes; and mCD45 represents murine-derived CD45+ cells.

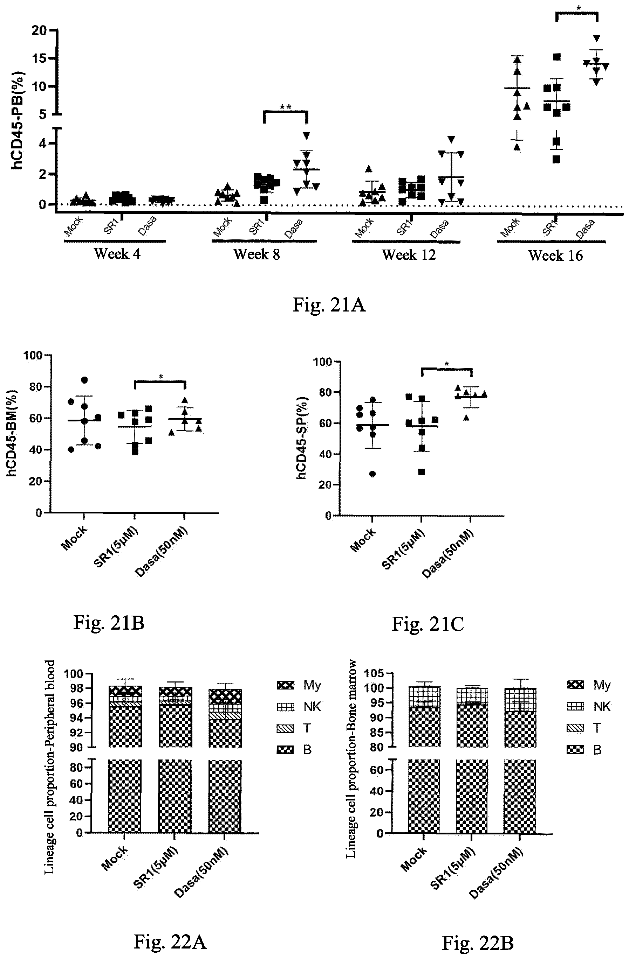

Fig. 21 shows that after 7 days of treatment with small-molecule: Mock (DMSO), SRI (5 µM), Dasatinib (50 nM) on cord blood-derived CD34+ cells, all cells are collected and transplanted into mice, the proportion of human-derived CD45 cells in peripheral blood of mice is detected by flow cytometry at week 4, 8, 12, and 16 after transplantation, and the proportion of human-derived CD45 cells in bone marrow and spleen of mice is detected by flow cytometry at week 16 after transplantation. In Fig. 21A, the abscissa represents the name of the inhibitor and the transplantation time, and the ordinate hCD45-PB (%) represents the proportion of human-derived CD45+ cells detected in the peripheral blood of mice. PB stands for peripheral blood, and hCD45 stands for human-derived CD45+ cells. In Fig. 21B, the abscissa represents the name of the inhibitor, and the ordinate hCD45-BM (%) represents the proportion of human-derived CD45+ cells detected in bone marrow of mice. BM stands for bone marrow, and hCD45 stands for human-derived CD45+ cells. In Fig. 21C, the abscissa represents the name of the inhibitor, and the ordinate hCD45-SP (%) represents the proportion of human-derived CD45+ cells detected in the mouse spleen. SP stands for spleen, and hCD45 stands for human-derived CD45+ cells.

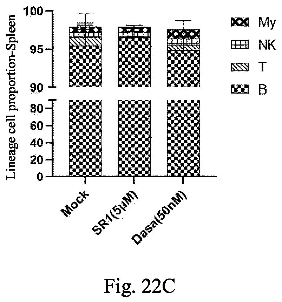

Fig. 22 shows that after 7 days of treatment with small molecule: Mock (DMSO), SRI (5 µM), Dasatinib (50 nM) on cord blood-derived CD34+ cells, all cells are collected and transplanted into mice, the proportion of human-derived hCD3+, hCD33+, hCD56+ and hCD19+ cells in peripheral blood, bone marrow, and spleen of mice are detected by flow cytometry at week 16 after transplantation, T, My, NK and B respectively represent human-derived T lymphocytes (T), myeloid cells (My), natural killer cells (NK), and B lymphocytes (B); wherein in Fig. 22A, the abscissa represents the name of the inhibitor, and the ordinate represents the proportion of hCD45+ cells of different lineages in peripheral blood; in Fig. 22B, the ordinate represents the proportion of hCD45+ cells of different lineages in bone marrow; and in Fig. 22C, the ordinate represents the proportion of hCD45+ cells of different lineages in spleen.

### EXAMPLES

### Example 1: Sorting CD34+HSCs from Umbilical Cord Blood for Subsequent Small Molecule Inhibitor Screening

Preparation of reagents: H-lyse Buffer (1×) solution, and Wash Buffer (1×) solution. 5ml of H-lyse Buffer (10×) stock solution (R&D, CAT. NO. WL1000) is taken, then adding 45ml of deionized water (Edigene, filtrated with 0.22µm filter membrane) to mix well to prepare an H-lyse Buffer (1×) solution. 5ml of Wash Buffer (10×) stock solution (R&D, CAT. NO. WL1000) is taken, then adding 45ml of deionized water to mix well to prepare a Wash Buffer (1×) solution.

Physiological saline is added to 10 ml of cord blood (Edigene) to a final volume of 30 ml. Human lymphocyte separation solution (Dakewe, CAT. NO. DKW-KLSH-0100) is added to the diluted blood, then centrifuging at 400g for 30min (setting acceleration speed 3, deceleration speed 0), sucking the buffy coat to centrifuge at 500g for 10min. The cell pellets are collected into a 50ml centrifuge tube, adding 10ml of H-lyse Buffer (1×) to lyse the red blood cells at room temperature for 10min. Then 10ml of Wash Buffer (1×) is added to stop the lysis reaction, adding physiological saline to make a final volume of 50ml. The above 50ml centrifuge tube is transferred to a high-speed centrifuge to centrifuge at 500g for 10min, discarding the supernatant, resuspending the cells with 50ml of physiological saline (1%HSA) to mix well, and taking 20µL of the cell suspension to a cell counter (Nexcelom, model: Cellometer K2), then transferring this centrifuge tube to high-speed centrifuge to centrifuge at 500g for 10min; discarding the supernatant, and adding the corresponding volume of magnetic beads (100ul FcR/1^{∗}10^8cells and 100ul CD34 MicroBeads/1^{∗}10^8cells) according to the counting result. The specific operations are as follows: firstly, FCR blocking reagent (Miltenyi biotec, Cat. No. 130-100-453, the amount of the reagent is determined according to the result of cell counting) is added to resuspend the cells, then adding premixed CD34 MicroBeads (CD34 MicroBead Kit UltraPure, human: MiltenyiBiotec, Cat. No. 130-100-453) to mix well to incubate in a refrigerator at 4°C for 30min. Physiological saline (1% HSA) is added to the centrifuge tube to a final volume of 50 ml, transferring to a high-speed centrifuge to centrifuge at 500 g for 10 min. A magnetic separator (MiltenyiBiotec, model: 130-042-102) and a magnetic stand (MiltenyiBiotec, model: 130-042-303) are provided, adjusting the magnetic separator to a suitable height, and putting it into the MS Column (MiltenyiBiotec, Cat. No. 130-042-201) or LS column (MiltenyiBiotec, Cat. No. 130-042-401) (the type of the Column should be selected according to the number of cells, please refer to the relevant product instructions for details), then placing a 15ml centrifuge tube (Corning, Cat. No. 430791) below to collect non-target cell suspension, rinsing the MS Column or LS Column with 1 ml (MS Column) or 3 ml (LS Column) of physiological saline (1% HSA). After centrifugation in the centrifuge tube in the above-mentioned high-speed centrifuge (Thermo, model: ST40), the supernatant is discarded, and the cells are resuspended in 1 ml (MS Column) or 3 ml (LS Column) of physiological saline (1% HSA), adding the cell suspension to each separation column (the amount of the separation column is determined according to the number of cord blood parts and the amount of cells); the centrifuge tube is washed with 1 ml (MS Column) or 3 ml (LS Column) of physiological saline (1% HSA), and the washing solution is added to the column.

The MS Column or LS Column is washed with 1 ml (MS Column) or 3 ml (LS Column) of physiological saline (1% HSA); repeating 3 times. The sorting column is transferred to the top of a new 15ml centrifuge tube, adding 2ml (MS Column) or 3ml (LS Column) of physiological saline (1%HSA) to elute the target cells, and then adding 1ml (MS Column) or 2ml (LS Column) of physiological saline (1% HSA) to elute the target cells once again. 20 µL of cell suspension is taken to count in a cell counter (Nexcelom, model: Cellometer K2), and the remaining cell suspension is centrifuged at 400 g for 5 min, incompletely discarding the supernatant to keep 1 ml of the supernatant, and resuspending the cells. A new MS Column is taken to add 1 ml of physiological saline (1% HSA) to rinse it, transferring the cell suspension of the resuspended cells to the MS Column, repeating the above washing and elution steps to obtain 3 ml of the target cell suspension. 20 µL of cell suspension is taken to count in a cell counter (Nexcelom, model: Cellometer K2), calculating the total number of cells according to cell density and cell suspension volume; the remaining cell suspension is centrifuged at 400 g for 5 min, discarding the supernatant for later use.

### Example 2: Concentration Testing and Screening of Small Molecule Inhibitor

A small molecule inhibitor stock solution is prepared according to the solubility and required solvent indicated in the instructions of the small molecule inhibitor product (see Table 1 for the Cat. Number of the small molecule inhibitor). Then the basal medium is prepared: SFEMII medium (stem cell, Cat. No. 09655) + 50ng/ml growth factor Flt-3L (PeProtech, Cat. No. 300-100UG) + 50ng/ml growth factor SCF (PeProtech, Cat. No. 300-07-100UG) + 50ng/ml growth factor TPO (PeProtech, Cat. No. 300-18-100UG) + 10ng/ml growth factor IL-6 (PeProtech, Cat. No. 200-06-20UG) + 1% double antibody (HyClone, Cat. No. sv30010). Cultural media containing different concentrations of a small molecule inhibitor are prepared according to the preset concentration gradient of the small molecule inhibitor by using the stock solution and the basal medium.

Firstly, the prepared medium is added to a 24-well plate (Corning, Cat. No. 3473), 950 µl per well, placing it in a carbon dioxide incubator (Thermo, Model: 3111) to preheat; the spare HSCs prepared in Example 1 are resuspended with SFEMII + 50ng/ml Flt-3L + 50ng/ml SCF + 50ng/ml TPO + 10ng/ml IL-6 + 1% double antibody, the volume of medium to be added is calculated according to 50µl cell suspension per well and 2^{∗}10^5/ml cell density per well. For example, the final volume of the cell culture medium per well is 1ml, the total number of cells per well is 2^{∗}10^5 cells calculated according to the cell density per well, and the density of 50µl of cell suspension added to each well is 4^{∗}10^6/ml, adjusting the density of the spare HSCs prepared in Example 1 to the calculated density of the cell suspension for addition; the preheated medium is taken out from the incubator, adding 50 µl of the cell suspension to each well, and after mixing well, observing the cell state under the microscope (OLYMPUS, model: CKX53), and then putting it into an incubator for culture.

**Table 1: Small Molecule Inhibitors**

| Cat. No. (The following are from Selleck) | Name of small molecule inhibitor | Action pathway / target |
|---|---|---|
| S1021 | Dasatinib (Dasa) | Src, c-kit, Abl |
| S2391 | Quercetin | Sirtuin, Src, PKC, PI3K |
| S8706 | UM-164 | Src, P38α/β |
| S2700 | KX2-391 | Src |
| S6500 | KX1-004 | Src |
| S1047 | SAHA | HDAC |
| S1999 | Sodium butyrate | HDAC |
| S3944 | Valproic acid (VPA) | HDAC |
| S7595 | Santacruzamate A(SIS3 Hcl) | HDAC |
| S7085 | IWP-2 | Porcn in the Wnt pathway |
| S7086 | IWR-1-endo | Wnt pathway |
| S8007 | VE821 | ATR |
| S7050 | AZ-20 | ATR |
| S7079 | SGC 0946 | DOT1L methyltransferase |
| S7353 | EPZ004777 | DOT1L methyltransferase |
| S7307 | GSK2606414 | EIF2AK3 (PERK, protein kinase R-like endoplasmic reticulum kinase) |
| S7400 | ISRIB (trans-isomer) | PERK |
| S2924 | CHIR-99021 (CT99021) HCl | GSK3α/β (glycogen synthetase kinase) |
| S2621 | AZD5438 | CDK1/2/9 (cyclin-dependent kinases1/2/9) |
| S4901 | JNK-IN-8 | JNK pathway |
| S7508 | JNK Inhibitor IX | JNK pathway |
| s7483 | DMOG | HIF prolyl hydroxylase |
| s1623 | Acetylcysteine | ROS (reactive oxygen species), TNFα (tumor necrosis factor) |
| S3114 | Vitamin C | ROS |
| s5433 | Sodium succinate | ROS |
| s2284 | Colchicine | microtubule polymerization |
| s2775 | Nocodazole | microtubule polymerization, Abl |
| S4505 | Vinblastine sulfate | microtubule formation, nAChR (subunit nicotinic acetylcholine receptor) |
| S3633 | Pyrrolidinedithiocarbamate ammonium | NF-κB |
| S4073 | Sodium 4-Aminosalicylate(S-4-A) | NF-κB |
| S1067 | SB431542 | TGFβRI/ALK5 |
| S1459 | Thiazovivin | ROCK (Rho-associated kinase) |
| S1049 | Y27632 | ROCK (Rho-associated kinase) |
| S1076 | SB203580 | P38 MAPK (mitogen-activated protein kinase) |
| S2449 | Forskolin | cAMP Activator |
| S7858 | Dibutyryl-cAMP (Bucladesine) | PKA (cAMP dependent protein kinase A) |
| s1907 | Metronidazole | DNA synthesis |
| S1992 | Fluticasone propionate(FLU) | Glucocorticoid receptor |
| s2250 | (-)-EpigallocatechinGallate | telomerase, DNA methyltransferase |
| s2923 | Salubrinal | eIF2α |
| s3925 | (-)-Epicatechingallate | VEGFR2 (vascular endothelial growth factor 2) |
| S4991 | Valpromide | VPA derivative, target unknown |
| S7120 | 3-deazaneplanocin A (DZNeP) HCl | S-adenosylhomocysteine hydrolase , Histone methyltransferase |
| S7315 | PFI-3 | PB1(5) bromodomains, SMARCA4/2A/2B |
| S7608 | UM171 | unknown |
| S8287 | CPI-455 HCl | KDM5 (histone demethylase) |
| s8615 | Sodium dichloroacetate (DCA) | PDK4/2 (pyruvate dehydrogenase kinase) |
| S5742 | Deferoxamine mesylate | unknown |
| S2858 | SR1 | AHR (aryl hydrocarbon receptor) |
| S4757 | Dihydrotestosterone(DHT) | Androgen receptor |
| S8280 | IMR-1 | Notch pathway |
| S2915 | GW9662 | PPARγ (peroxisome proliferatoractivated receptor) |
| S3003 | PGE2 | Wnt pathway |
| S1055 | Enzastaurin | PKC |
| S2911 | Go 6983 | PKC (protein kinase C) |
| s2776 | CPI-613 | PDH (pyruvate dehydrogenase), α-ketoglutarate dehydrogenase |

### Example 3: Flow Cytometry Detection of Stemness of HSCs and Maintenance of CD34+

The antibodies used in this example and their sources are shown in Table 2.

**Table 2: Antibodies**

| Antibody name | Manufactor | Cat. No. |
|---|---|---|
| APC/Cy7 anti-human CD45 | Biolegend | 304014 |
| APC anti-human CD38 | Biolegend | 356606 |
| Brilliant Violet 510™ anti-human CD34 | Biolegend | 343528 |
| PE anti-human CD90 (Thyl) | Biolegend | 328110 |
| FITC anti-human CD45RA | Biolegend | 304106 |
| APC Mouse IgG2a, κ Isotype Ctrl | Biolegend | 400220 |
| APC/Cyanine7 Mouse IgGl, κ Isotype Ctrl | Biolegend | 400128 |
| PE Mouse IgG2a, κ Isotype Ctrl | Biolegend | 400212 |
| FITC Mouse IgG2b, κ Isotype Ctrl | Biolegend | 402208 |
| Brilliant Violet 510™ Mouse IgG2a, κ Isotype Ctrl | Biolegend | 400268 |

20 µl of cells cultured for 6-7 days (D6-D7) in the above Example 2 is taken for counting, and a suspension of 2^{∗}10^5 cells is taken to add into a 1.5 ml centrifuge tube according to the counting result; centrifuging at 400 g for 5 min, and discarding the supernatant. 100 µl of PBS (phosphate buffered saline, HyClone, Cat. No. SH30256.01) containing 1% HSA (human serum albumin, Guangdong Shuanglin, Cat. No. S10970069) is taken to resuspend the cells, vortexing to mix well for later use. Then, a control cell sample is collected, the number of cells and the collection method are the same as those of the sample cells to be tested. The control cells are set as the NC group and the ISO group respectively, and the cells are selected from any sample or mixed cells of the samples to be tested in this batch of experiments, depending on the number of cells. In the same batch of experiments, each control group does not have repeated detection. See Table 3 for group settings.

**Table 3: Group settings**

| Group | Number of cells | Name of the added antibody | Antibody quantity |
|---|---|---|---|
| NC | 2×10^5 | - | - |
| ISO | 2×10^5 | APC Mouse IgG2a, κ Isotype Ctrl | 2µl |
| | | APC/Cyanine7 Mouse IgGl, κ Isotype Ctrl | 2µl |
| | | PE Mouse IgG2a, κ Isotype Ctrl | 2µl |
| | | FITC Mouse IgG2b, κ Isotype Ctrl | 2µl |
| | | Brilliant Violet 510™ Mouse IgG2a, κ Isotype Ctrl | 2µl |
| FMO38 | 2×10^5 | APC/Cy7 anti-human CD45 | 2µl |
| | | Brilliant Violet 510™ anti-human CD34 | 2µl |
| | | PE anti-human CD90 (Thyl) | 2µl |
| | | FITC anti-human CD45RA | 2µl |
| FMO90 | 2×10^5 | APC/Cy7 anti-human CD45 | 2µl |
| | | APC anti-human CD38 | 2µl |
| | | Brilliant Violet 510™ anti-human CD34 | 2µl |
| | | FITC anti-human CD45RA | 2µl |
| sample | 2×10^5 | APC/Cy7 anti-human CD45 | 2µl |
| | | APC anti-human CD38 | 2µl |
| | | Brilliant Violet 510™ anti-human CD34 | 2µl |
| | | PE anti-human CD90 (Thyl) | 2µl |
| | | FITC anti-human CD45RA | 2µl |

According to Table 3 above, antibodies are correspondingly added according to groups into the cell suspensions of the above-mentioned cell samples to be tested and control cell samples vortexing to mix well and incubate at room temperature for 15 min in the dark. After the 15 min incubation, 1 ml of PBS containing 1% HSA is added to each experimental sample to mix well, centrifuging at 400 g for 5 min at room temperature. After centrifugation, the supernatant is discarded, and the cells are resuspended in 100 µl of PBS containing 1% HSA for each experimental sample, storing the samples at room temperature away from light before testing. Flow cytometry is used to detect them.

The test results are analyzed as follows: 1) the target cell population is CD34+CD45+CD45RA-CD90+CD38- cell population; 2) the determination of the logic gate and gate position is shown in Fig. 1: firstly delineating the cell population, P1 gate; removing adherent cells in the cell population derived from the P1 gate, as the P2 gate; delineating CD34, CD45, CD45RA negative cell population in the cell population derived from the P2 gate by NC or ISO, as the Q3-LL gate (CD34-/CD45-), Q5-UL+Q5-LL gate (CD45RA-); delineating the CD90-negative cell population by FMO90, as the Q5-LL+Q5-LR gate; delineating the CD38-negative cell population by FMO38, as the Q6-LR gate; using the gates delineated by NC, ISO and FMO, it is determined that the cells delineated by Q3-UR-Q5-UL-Q6-LR gates are CD34 + CD45 + CD45RA-CD90 + CD38- target cells.

### Example 4: Single Molecule Screening

On the umbilical cord blood-derived CD34+ cells sorted in Example 1, the optimal concentrations of small molecule inhibitors and the ability to maintain the sternness of HSCs are screened according to the same method as in Example 2. After 6-7 days of small molecule induction, the expression of cell surface markers (CD34+CD45+CD90+CD45RA-CD38-) of long-term hematopoietic stem cells (LT-HSCs) is detected by flow cytometry according to the same method as in Example 3.

A total of five rounds of screening are carried out in this example, and the inhibitors and test concentrations for each round of screening are shown in Table 4, Table 5, Table 6, Table 7, and Table 8, and the results are shown in Figs. 2-6, respectively.

The results in Fig. 2 show that, SAHA is significantly better than other inhibitors in Table 4 in increasing the proportion of LT-HSCs, and it is 2-20 times more effective than other inhibitors. When SAHA is 5 µM, the cell state is poor under a microscope, and the proliferation is poor by cell counting comparison, so a concentration of 1 µM is selected for subsequent screening. SAHA (1 µM) is slightly better than the control group (Mock, 0.01% DMSO) in maintaining the proportion of CD34+ cells.

The results in Fig. 3 show that, compare with the small molecule inhibitors UM171, PGE2, SRI, GW9662 and FLU reported in the known literatures in Table 5 (Fares I, et al. Science.2014; Evans T. Cell Stem Cell. 2009; Boitano A E, et al.Science.2010; Guo B, et al. Nature Medicine. 2018; Guo B, et al. Nature Medicine. 2017), SAHA is 2-20 times more effective than the reported small molecules in maintaining sternness of HSCs; and in terms of maintaining the proportion of CD34+ cells, there is no significant difference between SAHA and SRI, but SAHA is significantly better than UM171, PGE2, GW9662, and FLU.

The results in Fig. 4 show that, SAHA is about 2-15-fold higher than other inhibitors in Table 6 in maintaining sternness of HSCs. At the same time, SAHA is better than other inhibitors in maintaining the proportion of CD34+ cells.

The results in Fig. 5 show that, SAHA is significantly better than other inhibitors in Table 7 in maintaining the sternness of HSCs, and is 1-12 times more effective than other inhibitors; followed by Enzastaurin used at low concentrations, the proportion of LT-HSCs is 3-10 times higher than that of other inhibitors (other than SAHA). In terms of maintaining the proportion of CD34+ cells, JNK-IN-8 can maintain the proportion of CD34+ cells at about 90%; SAHA and Dasatinib are slightly better than other inhibitors except JNK-IN-8.

The results in Fig. 6 show that, in terms of maintaining the sternness of LT-HSCs, the effect pf Valproic acid (VPA) in Table 8 is significantly higher than other small molecules by about 30 times, and higher than SAHA by about 1 time; SAHA is 15 times more effective than other small molecules (except VPA). SAHA and VPA are superior to other small molecules in maintaining the proportion of CD34+ cells.

To sum up: in this Example, a total of 5 small molecules that can maintain the sternness of LT-HSCs and a high proportion of CD34+ cells are screened out; and they are respectively VPA and SAHA which target HDAC, Dasatinib targeting Src., Enzastaurin targeting PKC, and JNK-IN-8 targeting JNK.

**Table 4: First Round of Screening**

| Name of small molecule inhibitor | Test concentration |
|---|---|
| VE821 | 0.1µM, 1µM, 5µM, 10µM |
| AZ20 | 0.1µM, 1µM, 5µM, 10µM |
| PFI-3 | 0.2µM, 2µM, 5µM, 10µM |
| Sodium 4-Aminosalicylate (S-4-A) | 0.1mM, 1mM, 5mM, 10mM |
| PDTC | 1nM, 5nM, 10nM, 50nM |
| SAHA | 0.1µM, 1µM, 5µM, 10µM |
| Santacruzamate A(SIS3 HCL) | 0.1µM, 1µM, 5µM, 10µM |
| SR1 | 0.1µM, 1µM, 5µM, 10µM |

**Table 5: Second Round of Screening**

| Name of small molecule inhibitor | Test concentration |
|---|---|
| SAHA | 1µM |
| SR1 | 5µM |
| UM171 | 350nM |
| PGE2 | 10µM |
| GW9662 | 1µM |
| FLU | 1µM |

**Table 6: Third Round of Screening**

| Name of small molecule inhibitor | Test concentration |
|---|---|
| SAHA | 1µM |
| Vitamin C | 5µg/ml, 25µg/ml, 50µg/ml |
| EPZ004777 | 0.5µM, 5µM, 10µM |
| Forskolin | 5µM, 10µM, 20µM |
| CPI-455 | 1µM, 5µM, 10µM |
| DZNeP | 10nM, 50nM, 250nM |
| CHIR-99021 | 1µM, 3µM, 10µM |
| Butyrate | 50µM, 250µM,500µM, |
| SB203580 | 1µM, 5µM, 10µM |
| IWP-2 | 1µM, 5µM, 10µM |
| IWR-1-endo | 1µM, 5µM, 10µM |
| JNK-inhibitor iX | 1µM, 5µM, 10µM |
| Dibutyryl-cAMP | 1µM, 5µM, 10µM |
| Thiazovivin | 1µM, 5µM, 10µM |
| IMR-1 | 1µM, 5µM, 10µM |
| SB431542 | 1µM, 5µM, 10µM |
| Quercetin | 0.5µM, 1µM, 5µM |

**Table 7: Fourth Round of Screening**

| Name of small molecule inhibitor | Test concentration |
|---|---|
| SAHA | 1µM |
| Dasatinib | 0.5µM 5µM 10µM |
| SGC0496 | 1µM 5µM 10µM |
| JNK-IN-8 | 1µM 2µM 10µM |
| Enzastaurin(LY317615) | 1µM 5µM 10µM |

**Table 8: Fifth Round of Screening**

| Name of small molecule inhibitor | Test concentration |
|---|---|
| SAHA | 1µM |
| Valproic acid | 100µM 500µM 1000µM |
| Valpromide | 100µM 500µM 1000µM |
| AZD5438 | 1µM 5µM 10µM |
| Go 6983 | 1µM 5µM 10µM |
| Sodium succinate | 5µM 10µM 50µM |
| Sodium dichloroacetate (DCA) | 0.1mM 1mM 5mM |
| Salubrinal | 1µM 5µM 10µM |
| CPI-613 | 5µM 10µM 50µM |
| ISRIB(trans-isomer) | 1µM 5µM 10µM |
| Acetylcysteine | 1µM 5µM 10µM |
| GSK2606414 | 10nM 50nM 100nM |
| DMOG | 1µM 5µM 10µM |
| Metronidazole | 1µM 5µM 10µM |
| DHT | InM 5nM 50nM |
| Nocodazole | 1µM 5µM 10µM |
| Vinblastine sulfate | 1µM 5µM 10µM |
| Epigallocatechin Gallate | 1µM 5µM 10µM |
| Deferoxamine mesylate: | 10nM 50nM 500nM |
| Epicatechin gallate | 10nM 50nM 500nM |
| Colchicine | 50nM 500nM 1000nM |
| Y27632 | 5µM 10µM 20µM |

### Example 5: Screening of the Combination of Two Small Molecule Inhibitors

On the umbilical cord blood-derived CD34+ cells sorted in Example 1, the screening of the best combination of two small molecule inhibitors to maintain the sternness of HSCs is carried out according to the same method as in Example 2. After 6-7 days of induction with a combination of small molecule inhibitors, the expression of LT-HSCs cell surface markers (CD34+CD45+CD90+CD45RA-CD38-) is detected by flow cytometry according to the same method as in Example 3.

The small molecule SAHA that can significantly maintain the sternness of HSCs screened according to the above Example 4 is respectively combined with other inhibitors. The specific combination and its concentration are shown in the relevant drawings, and the results are shown in Figs. 7-11, respectively.

The results in Fig. 7 show that, in terms of maintaining the sternness of HSCs and the proportion of CD34+ cells, the combinations of SAHA and 4 inhibitors of the cell signaling pathways in Table 4 which are associated with hematopoietic stem cell expansion, i.e., SAHA+SR1, SAHA+VE821, SAHA+PFI-3, and SAHA+S-4-A, are not significantly better than SAHA alone.

The results in Fig. 8 show that, in terms of maintaining the sternness of HSCs, each of the inhibitors reported in the aforementioned literatures, namely the inhibitors SRI, UM171, PGE2, GW9662, and FLU in Table 5, is far less effective than SAHA when used alone, and the combination of each of them with SAHA is also less effective than SAHA alone. There is also no significant difference between SAHA alone and a combination of each of these inhibitors and SAHA in maintaining the proportion of CD34+ cells.

The results in Fig. 9 show that, in terms of maintaining the sternness of HSCs, in the combination of SAHA and Butyrate in Table 6, the effect of low concentration of Butyrate is not as good as that of SAHA alone; and the effect of high concentration of Butyrate is better than that of SAHA alone, but under the condition of high concentration, Butyrate is highly toxic to cells; and the results obtained in Example 4 show that, the effect of Butyrate alone is not as good as that of SAHA alone, and Butyrate is the same target inhibitor as SAHA, so further research on Butyrate will not be conducted in the future. SAHA is respectively combined with EPZ004777 and DZNeP in Table 6, and the combinations, i.e., SAHA+EPZ004777 and SAHA+DZNeP, are better than SAHA alone. These combinations of two small molecule inhibitors are not significantly different from SAHA alone in maintaining the proportion of CD34+ cells. Therefore, the result of the screening shows that the combinations of SAHA+EPZ004777 and SAHA+DZNeP well maintain the sternness of HSCs and the proportion of CD34+ cells.

The results in Fig. 10 show that, in terms of maintaining the sternness of HSCs, as for the combinations of SAHA with each of the 4 small molecule inhibitors in Table 7, the combinations of SAHA+Dasatinib and SAHA+JNK-IN-8 are 3 times more effective than SAHA alone, 30 times more effective than the Mock group. In terms of maintaining the proportion of CD34+ cells, the combinations of SAHA+Dasatinib and SAHA+JNK-IN-8 have no significant difference with SAHA alone, which are 5%-10% higher than Mock group. A combination of the small molecule Enzastaurin (which can better maintain the sternness of LT-HSCs in single-molecule screening) with SAHA is much less effective than SAHA alone, thus further research on the combination of Enzastaurin and SAHA is not conducted. Therefore, the result of the screening shows that, the combinations of SAHA+Dasatinib and SAHA+JNK-IN-8 are more effective in maintaining the sternness of HSCs and the proportion of CD34+ cells.

The results in Fig. 11 show that, in terms of maintaining the sternness, as for the combination of SAHA with VPA in Table 8, the effect of the combination SAHA+VPA is higher than SAHA alone by about 2-4 times, and higher than Mock group by 20 times. In terms of maintaining the proportion of CD34+ cells, the combination SAHA+VPA has no significant difference with SAHA alone, and the effect is higher than the Mock group by 10%-20%.

In summary, in the screening of the combination of two small molecule inhibitors, the combinations that can maintain the sternness of LT-HSCs and the proportion of CD34+ cells are screened out as follows: SAHA+Dasatinib, SAHA+DZNeP, SAHA+EPZ004777, SAHA+JNK-IN-8, and SAHA+VPA.

### Example 6: Screening of the Combination of Three Small Molecule Inhibitors

On the umbilical cord blood-derived CD34+ cells sorted in Example 1, the screening of the best combination of three small molecule inhibitors to maintain the sternness of HSCs is performed according to the same method as in Example 2. After 6-7 days of induction with a combination of small molecule inhibitors, the expression of LT-HSCs cell surface markers (CD34+CD45+CD90+CD45RA-CD38-) is detected by flow cytometry according to the same method as in Example 3.
(1) The combinations of SAHA+EPZ004777 and SAHA+DZNeP screened in Example 5 (which can maintain the sternness of HSCs) are subjected to the combination of three small molecule inhibitors, and the results are shown in Fig. 12.

The results in Fig. 12 show that, in terms of maintaining the sternness of HSCs, the combination of three small molecule inhibitors, i.e., SAHA+EPZ004777+DZNeP, is about 20 times more effective than the Mock group, is about 2 times more effective than SAHA alone and the combination of two small molecule inhibitors, i.e., SAHA+EPZ004777, and is slightly more effective than that of the combination of two small molecule inhibitors, i.e., SAHA+DZNeP. In terms of maintaining the proportion of CD34+ cells, the combination of three small molecule inhibitors and the combination of two small molecule inhibitors have significant differences with the Mock group, and the proportion of CD34+ cells is about 80%.

(2) The combinations of SAHA+EPZ004777, SAHA+DZNeP, SAHA+JNK-IN-8 and SAHA+Dasatinib screened in Example 5 (which can maintain the sternness of HSCs) are subjected to the combination of three small molecule inhibitors, and the results are shown in Fig. 13 and Fig. 14, respectively.

The results in Fig. 13 show that, in terms of maintaining the sternness of HSCs, the combination of SAHA+JNK-IN8 is not as effective as SAHA+Dasatinib, thus further research on SAHA+JNK-IN8 will not be conducted. There is no significant difference between SAHA+Dasatinib+EPZ004777 and SAHA+Dasatinib, and other combinations of three small molecule inhibitors are not as effective as SAHA+Dasatinib in maintaining the sternness of LT-HSCs. In terms of maintaining the proportion of CD34+ cells, SAHA+Dasatinib maintains the proportion of CD34+ cells at about 80%.

The results in Fig. 14 show that, the combinations of two small molecule inhibitors, i.e., SAHA+Dasatinib and SAHA+VPA, screened in Example 5 (which can maintain the sternness of HSCs) are recombinated. In terms of maintaining the sternness of HSCs, the combination of three small molecule inhibitors, i.e., SAHA+VPA+Dasatinib, is 50 times more effective than Mock group and 1.5-2 times more effective than the combination of two small molecule inhibitors. In terms of maintaining the proportion of CD34+ cells, the effect of the combination of three small molecule inhibitors, i.e., SAHA+VPA+Dasatinib, is higher than that of the Mock group by 20%.

To sum up, in terms of maintaining the sternness of LT-HSCs, most of the combinations of three small molecule inhibitors are not as effective as the combination of two small molecule inhibitors, i.e., SAHA+Dasatinib; while among the combinations of three small molecule inhibitors, the best combinations are SAHA+EPZ004777+DZNeP, and SAHA+Dasa+VPA.

### Example 7: Comparison between an Inhibitor Used Alone and Inhibitors Used in Combination for the Screened Inhibitors SAHA, VPA, and Dasatinib and the Inhibitors UM171 and SR1 Reported in the Literatures

On the umbilical cord blood-derived CD34+ cells sorted in Example 1, according to the same method as Example 2, comparison between an inhibitor used alone and inhibitors used in combination for the screened inhibitors SAHA, VPA, and Dasatinib and the inhibitors UM171 and SRI reported in the literatures (Fares I, et al. Science.2014; Boitano A E, et al. Science.2010;) is performed. After 6-7 days of induction with small molecule inhibitor(s), the expression of LT-HSCs cell surface markers (CD34+CD45+CD90+CD45RA-CD38-) is detected by flow cytometry according to the same method as in Example 3; and the results are shown in Fig. 15.

The results in Fig. 15 show that, in terms of maintaining the sternness of HSCs, when the small molecules are used alone, the effect of SAHA or VPA is higher than that of SRI or UM171 by 2-5 times. In the combinations of two small molecule inhibitors, the effect of SAHA+Dasatinib or VPA+Dasatinib is higher than that of SAHA+SR1 or SAHA+UM171 by 1.5-2 times. The effect of the combination of three small molecule inhibitors, i.e., SAHA+DZNeP+EPZ004777, is slightly lower than that of SAHA+Dasatinib and VPA+Dasatinib. In terms of maintaining the proportion of CD34+ cells, there is no significant difference between a single small molecule inhibitor SAHA or VPA and the combination of two small molecule inhibitors, i.e., SAHA+Dasatinib or VPA+Dasatinib, and the proportion of CD34+ cells is all about 80%.

To sum up, in terms of maintaining the sternness of LT-HSCs and the proportion of CD34+ cells, the combination of two small molecule inhibitors, i.e., SAHA+Dasatinib or VPA+Dasatinib, is more effective than the small molecule inhibitor used alone, the combination of two small molecule inhibitors, i.e., SAHA+SR1 or SAHA+UM171, and the combination of three small molecule inhibitors, i.e., SAHA+DZNeP+EPZ004777.

### Example 8: CD34+ Hematopoietic Stem Cell Colony Formation and Culture

In this example, qualitative and quantitative detections are performed by using colony-forming unit (CFU) to detect the in vitro function of cord blood-derived hematopoietic stem cells after induction with a small molecule inhibitor, thereby verifying the in vitro differentiation potential.

Firstly, 100 mL of MethoCult^{™} H4034 Optimum (stem cell, Cat. No. 04034) is aliquoted, then thawing at 2-8°C overnight; shaking vigorously for 1-2 min and standing for 10 min until the bubbles rise to the liquid level. After tightly fitting the 50mL syringe needle to the 5mL disposable syringe, the medium is aspirated to 1mL, pushing out the syringe completely to exhaust the gas in the syringe, and re-absorbing 3mL into each 15mL centrifuge tube (Corning, Cat. No. 430791); storing at 2-8°C for 1 month and at -20°C for a long time. Do not freeze and thaw repeatedly.
3 mL of medium MethoCult^{™} H4034 Optimum is prepared then thawing overnight at room temperature (15-25°C) or 2-8°C.

Cell seeding is performed. The cell suspension undergoing 7 days of expansion and culture after induction with small molecule inhibitor(s) (cord blood-derived CD34+ hematopoietic stem cells after induction with small molecule inhibitor(s)) is taken for cell counting, then aspirating the cell suspension at 100 times the seeding density according to the counting results (for example, the seeding density is 100 cells/well/3ml, and 10000 cells should be collected), adding to 1ml of 2% FBS (Gibco, Cat. No. 16000-044)-IMDM (Gibco, Cat. No. 12440-053) medium to mix well for use. After mixing the above cells, 50 µl of the cell suspension is aspirated into 0.5 mL of IMDM (2% FBS) to resuspend the cells (equivalent to 10-fold dilution of the cell suspension). After mixing well, 100 µl of the cell suspension (100 cells) is taken to add into 3mL of MethoCult^{™}H4034 Optimum; vortexing for at least 4s and standing for 10min until the bubbles rise to the liquid level. 3 cc Syringes (Stem cell, Cat. No. 28240) is used in conjunction with Blunt-End Needles 16 Gauge (Stemcell, Cat. No. 28110) to aspirate the obtained cell suspension to 1 mL, then pushing it out of the syringe to exhaust the gas in the syringe, and re-absorbing all the obtained cells suspension to inject 3mL into one well of SmsrtDishTM-6 (stem cell, Cat. No. 27370, 6-well plate), and slowly tilting the 6-well plate so that the cell suspension evenly covers the bottom of the well. After inoculating all cells as described above, 3 ml of sterile PBS is added to gaps between wells of the 6-well plate to prevent the medium from drying up. The 6-well plate is covered with a lid, then placing the plate in a carbon dioxide incubator (Thermo, model: 3111) to culture for 14 days at 37°C., 5% CO₂, and 95% relative humidity.

Colonies are observed on day 7 and day 14 of the culture, and colonies are counted with a STEMgridTM-6 counting grid (stem cell, Cat. No. 27000) after culturing for 14 days. The criteria for determining colonies are as follows (different types of colonies can reflect the ability of HSCs to form colonies and maintain the sternness):
CFU-GEMM (CFU-G, CFU-E, CFU-MM): granulocyte-erythrocyte-macrophage-megakaryocyte colony forming unit. A colony contains erythrocytes and 20 or more non-erythrocytes (granulocytes, macrophages, and/or megakaryocytes), usually the erythrocytes are located in the center of the colony and surrounded by non-erythrocytes, and non-erythrocytes can also be concentrated on one side of the erythrocytes. Generally, colonies of CFU-GEMM are larger than those of CFU-GM or BFU-E and they rare in most cell samples (usually 10% of the total colonies).
CFU-GM: a colony contains more than 20 granulocytes (CFU-G) and/or macrophages (CFU-M). Without appearing red or brown, individual cells within a colony are often distinguishable, especially at the edge of the colony, and a large colony may have one or more dense dark nuclei. Erythropoietin (EPO) is not required for the growth and differentiation of this colony.
BFU-E: burst erythrocyte colony-forming unit, forming colonies consist of single or multiple cell clusters, each colony containing >200 mature erythrocytes. When cells are hemoglobinated they appear red or brown, making it difficult to distinguish individual cells within each cluster, BFU-E are more immature progenitor cells whose growth require erythropoietin (EPO) and other cytokines, especially interleukin 3 (IL-3) and stem cell factor (SCF) for optimal growth of their colonies.
CFU-E: erythrocyte colony-forming unit, which can form 1-2 cell clusters containing 8-200 red blood cells, when the cells are hemoglobinized they appear red or brown, making it difficult to distinguish individual cells within the colony. CFU-E are progenitors of the mature erythroid lineage, and they require erythropoietin (EPO) to promote their differentiation.

### Example 9: Comparison of In Vitro Clonogenic Ability between an Inhibitor Used Alone and Inhibitors Used in Combination for the Screened Inhibitors SAHA, VPA, and Dasatinib and the Inhibitors UM171 and SR1 Reported in the Literatures

The Comparison of in vitro clonogenic ability between an Inhibitor Used Alone and Inhibitors Used in Combination for the screened inhibitors SAHA, VPA, and Dasatinib and the inhibitors UM171 and SRI reported in the literatures is carried out on the umbilical cord blood-derived CD34+ cells sorted in Example 1. In vitro clone (CFU) formation is detected by the same method as in Example 8 after inducing the cells with small molecule inhibitors for 7 days, and the number of clones is counted 14 days after inoculation of the cells, and the CFU-GEMM is analyzed. The results are shown in Fig. 16, wherein BFU-E, CFU-E, CFU-GM, and CFU-GEMM represent clones of different blood lineages such as erythroid, myeloid, and lymphoid.

The results in Fig. 16 show that, in terms of the total number of clones, the combination VPA+Dasatinib is significantly more effective than other combinations. In terms of the number of GEMM clones formed by the differentiation of LT-HSCs, the combination VPA+Dasatinib is significantly more effective than the small molecular inhibitors reported in the known literatures (Fares I, et al. Science.2014; Boitano A E, et al. Science.2010; ) when an inhibitor is used alone or used in combination with SAHA (i.e., more effective than SRI, UM171, SAHA+SR1, SAHA+UM171). In terms of the number of total clones or GEMM clones, SAHA+Dasatinib is more effective than VPA+Dasatinib, and SAHA+DZNeP+EPZ004777 is far inferior to VPA+Dasatinib and SAHA+Dasatinib.

In conclusion, in terms of in vitro clonogenic ability, the combinations of VPA+Dasatinib and SAHA+Dasatinib are superior to the small molecule SRI, UM171 reported in the known literatures when an inhibitor is used alone or used in combination with SAHA.

### Example 10: Verification of the Effect of Src Pathway Inhibitors

On the umbilical cord blood-derived CD34+ cells sorted in Example 1, other small molecule inhibitors targeting Src are screened according to the same method as in Example 2. After 6-7 days of induction with a combination of small molecule inhibitors, the expression of LT-HSCs cell surface markers (CD34+CD45+CD90+CD45RA-CD38-) is detected by flow cytometry according to the same method as in Example 3. The results are shown in Fig. 17, wherein the small molecule inhibitors and concentrations screened in this round are shown in Table 9.

**Table 9: Src pathway inhibitors and selected concentrations**

| Name of small the molecule inhibitor | Tested concentration |
|---|---|
| SAHA | 1µM |
| UM-164 | 1µM, 5µM, 10µM |
| KX2-391 | 10nM, 50nM, 100nM |
| KX1-004 | 10µM, 50µM, 100µM |

The results in Fig. 17 show that, in terms of maintaining the sternness of LT-HSCs, inhibitor UM-164 targeting Scr is 5 times more effective than SAHA, and the effect of other inhibitor KX1-004 targeting Scr is not significantly different from that of SAHA. In terms of maintaining the proportion of CD34+ cells, there is no significant difference between UM164 and SAHA, and the proportion of CD34+ cells is maintained at about 80%. The above results indicate that, Src as a new target plays an important role in maintaining the sternness of HSCs. Combined with inhibitors targeting HDAC, the effect of inhibitors targeting Scr is further enhanced in maintaining HSCs sternness and promoting HSCs expansion.

### Example 11: Comparison of In Vitro Expansion and the Ability to Maintain the Stemness of Hematopoietic Stem Cells for the Screened Inhibitor Dasatinib and the Inhibitors UM171 and SR1 Reported in the Literatures

On the umbilical cord blood-derived CD34+ cells sorted in Example 1, a comparison of in vitro expansion and the ability to maintain the sternness of hematopoietic stem cells for the screened inhibitor Dasatinib and the inhibitors UM171 and SRI reported in the literatures is carried out. After 6-8 days of induction with the small molecule inhibitor, the expression of LT-HSCs cell surface markers (CD34+CD45+CD90+CD45RA-CD38-) is detected by flow cytometry according to the same method as in Example 3. 20 µL of cell suspension is respectively taken on day 2, day 4, day 6 and day 8 of the culture to count in a cell counter (Nexcelom, model: Cellometer K2), calculating the final absolute number of CD34+ cells and LT cells on day 8 (absolute number of cells = cell proportion ^{∗}total number of cells), the results are shown in Fig. 18.

The results in Fig. 18 show that, in terms of maintaining sternness, Dasatinib is significantly better than SRI and UM171, particularly Dasatinib is about 1.2 times more effective than SRI and about 2.8 times more effective than UM171. The absolute number of LT cells in the Dasatinib group is not significantly better than that in the SRI group, and is similar to that in the UM171 group. In terms of maintaining the proportion of CD34+ cells, the proportion of CD34+ cells is maintained at about 40% after treating with Dasatinib for 8 days, while that is maintained at about 65% in the SRI group, and that is maintained at about 40% in the UM171 group. Therefore, the absolute number of CD34+ cells in the Dasatinib group is not significantly better than that in the SRI group and the UM171 group.

### Example 12: Validation of the Effect on In Vivo Transplantation of Hematopoietic Stem Cells for the Screened Inhibitor Dasatinib and the Inhibitor SR1 Reported in the Literatures

**On the umbilical cord blood-derived CD34+ cells sorted in Example 1, a comparison of the in** vivo hematopoietic system reconstitution ability between the screened small molecule inhibitor Dasatinib and the inhibitor SRI reported in the literature which are used alone is performed. The concentrations and groups of small molecule inhibitors used in this Example are shown in Table 10.

**Table 10. Small molecule inhibitor concentrations**

| Group | Concentration of small molecule inhibitor |
|---|---|
| Mock | - |
| SRI | 5µM |
| Dasatinib | 50nM |

Preparation of cell culture medium: as for SFEMII medium + 50ng/ml growth factor Flt-3L + 50ng/ml growth factor SCF + 50ng/ml growth factor TPO + 10ng/ml growth factor IL-6 + 1% double antibody, catalog numbers of the used medium, growth factors, and double antibodies, etc. are the same as those described in Example 2. Different small molecule inhibitors are respectively added according to the groups set in Table 10.

The prepared cell culture medium is added to a 24-well plate, 950 µl per well, then placing it in a carbon dioxide incubator to preheat; the prepared HSCs in Example 1 are resuspended with SFEMII + 50ng/ml Flt-3L + 50ng/ml SCF + 50ng/ml TPO + 10ng/ml IL-6 + 1% double antibody, then calculating the volume of the added medium according to 50ul of cell suspension per well, and the cell density per well of 1^{∗}10^5/ml; taking out the preheated medium from the incubator, adding 50 µl of cell suspension to each well to mix well, and observing the cell state under a microscope, and then putting it into an incubator for culture. The amount of initial cultured cells for transplantation in each mouse is 1^{∗}10^5/mouse, and the cells expanded in each well of the 24-well plate is sufficient for transplantation into one mouse. During the cell culture process, cells are counted every other day, the counting method and cell counter are the same as in Example 1, making sure that the cell density does not exceed 8^{∗}10^5/ml. If the cells are too dense, the cells in a well should be divided in time and fresh medium is added.

The expression of LT-HSCs cell surface markers (CD34+CD45+CD90+CD45RA-CD38-) is detected according to the same method as in Example 3 after treating the cells with the small molecule inhibitor for 7 days.

Mice are prepared, with 8 mice per group. Mice are purchased from Beijing Weitongda Biotechnology Co., Ltd., the strain is NPG (NOD-Prkdc^{scid}112rg^{null}/Vst), 6-week-old female mice, and the weight difference between the mice is controlled within 3 g. The mice are irradiated with a half-lethal dose before cell transplantation, and the irradiation dose is 1.6 Gy.

The cultured cell suspension (the initial culture cell amount is 1^{∗}10^5/ml) are collected to centrifuge at 400g for 5min, discarding the supernatant, then resuspending the cells with 100µl of physiological saline (1%HSA), and injecting the cells into an irradiated NPG mice through the tail vein, different groups of mice are labeled.

After the cells are transplanted into the mice, the peripheral blood of the mice is respectively collected at week 4, week 8, and week 12, and the proportion of human CD45 is detected by flow cytometry; the mice are sacrificed at week 16, and the peripheral blood, bone marrow cells and spleen cells of the mice are collected to detect the proportion of human CD45, human CD19, human CD3, human CD33 and human CD56 by flow cytometry. Antibodies and 7-AAD dye used in this example and their sources are shown in Table 11.

**Table 11: Antibodies and 7-AAD**

| Antibody name | Manufacture | Catalog number |
|---|---|---|
| FITC anti-mouse CD45 | Biolegend | 103108 |
| APC/Cy7 anti-human CD45 | Biolegend | 304014 |
| Brilliant Violet 510^{™} anti-human CD3 | Biolegend | 300448 |
| PE anti-human CD19 | Biolegend | 363004 |
| Brilliant Violet 421^{™} anti-human CD33 | Biolegend | 303416 |
| APC anti-human CD56 | Biolegend | 304610 |
| 7-AAD Viability Staining Solution | Biolegend | 420404 |

The proportion of human CD45 in peripheral blood of mice is detected by flow cytometry, and the set cell detection groups are shown in Table 12.

**Table 12:**

| Group | Number of cells | Name of the added antibody | Amount of the antibody |
|---|---|---|---|
| NC | 2×10^5 | -- | -- |
| Sample | 2×10^5 | APC/Cy7 anti-human CD45 | 2µl |
| | | FITC anti-mouse CD45 | 2µl |

Preparation of IX erythrocyte lysate: 5ml of RBC Lysis/Fixation Solution 10X stock solution (Biolegend, Cat. No. 422401) is taken, adding 45ml of deionized water (Edigene, iltered with a 0.22µm filter) to mix well to prepare IX erythrocyte lysate.

The peripheral blood of mice (about 100 µl) is collected, and antibodies are added according to the groups set in Table 12, vortexing to mix well and incubating at room temperature for 15 min in the dark. After the incubation is completed, 1.2 ml of IX red blood cell lysis solution is respectively added to the NC and each sample, vortexing to mix well, and lysing at room temperature for 15 min in the dark, during this period the sample centrifuge tube is inverted every 3 min. After the lysis is completed, the sample solutions are centrifuged at 400 g for 5 min at room temperature. After the centrifugation, the supernatant is discarded, adding 1 ml of PBS containing 1% HSA to each experimental sample to mix well, centrifuging at 400 g for 5 min at room temperature. After centrifugation, the supernatant is discarded, adding 100 µl of PBS containing 1% HSA and 5 µl of 7-AAD dye to each experimental sample to mix well by vortexing, and incubating at room temperature for 5 min in the dark. After the incubation, 1 ml of PBS containing 1% HSA is added to the NC and each sample to mix well, centrifuging at 400 g for 5 min at room temperature. After the centrifugation, the supernatant is discarded, then adding 100ul of PBS containing 1% HSA to each experimental sample to resuspend the cells; the samples are stored at room temperature in the dark before testing, and detected by flow cytometry.

The test results are analyzed as follows: 1) the target cell population is human CD45+ cell population; 2) the determination of the logic gate and gate position is shown in Fig. 19: firstly, the cell population is delineated as the P1 gate; the adherent cells are removed in the cell population derived from the P1 gate as the P2 gate; the viable cell population in the cell population derived from P2 gate is delineated with 7-AAD as P3 gate; mouse CD45- and human CD45- cell population in the cell population derived from P3 gate (Q2-LL gate) is delineated by NC; the gate delineated by NC is used to determine that the cells delineated by the Q2-UL gate are human CD45+ target cells. The transplantation efficiency of human hematopoietic stem cells is expressed by the proportion of human CD45 cells, and the calculation method is as follows: human CD45% / (human CD45%+mouse CD45%).

The proportions of human CD45, human CD19, human CD3, human CD33 and human CD56 in peripheral blood, bone marrow cells and spleen cells of mice are detected by flow cytometry. See Table 13 for the set cell detection groups.

**Table 13:**

| Group | Number of cells | Name of the added antibody | Amount of the antibody |
|---|---|---|---|
| NC | 2×10^5 | -- | -- |
| Sample | 2×10^5 | APC/Cy7 anti-human CD45 | 2µl |
| | | FITC anti-mouse CD45 | 2µl |
| | | Brilliant Violet 510^{™} anti-human CD3 | 2µl |
| | | PE anti-human CD19 | 2µl |
| | | Brilliant Violet 421^{™} anti-human CD33 | 2µl |
| | | APC anti-human CD56 | 2µl |

The peripheral blood of mice (about 100 µl) is collected, and antibodies are respectively added according to the groups set in Table 13. Subsequent blood sample processing is consistent with the above-mentioned operation for detecting the proportion of human CD45 in peripheral blood of mice. After the operation, a flow cytometer is used for detection.

The mice are sacrificed by cervical dislocation, and the tibia and femur of one hind leg of the mice are taken. Ophthalmic scissors and ophthalmic forceps are used to cut off both ends of the tibia and femur respectively to expose the marrow cavity. 1 ml syringe is used to draw the pre-cooled PBS containing 1% HSA, the needle is injected into one end of the bone marrow cavity, and the PBS is injected forcefully to flush out the bone marrow cells from the other end of the bone marrow cavity. Tibial and femoral marrow cavities are respectively rinsed with 2 ml of PBS. The cell suspension of bone marrow is repeatedly blown and sucked with a pipette, then filtering through a 40um cell mesh (BD, catalog number: 352340), centrifuging at 400 g for 5 min at room temperature. After centrifugation, the supernatant is discarded, and the bone marrow cells are used for later use.

The mice are sacrificed by cervical dislocation, and the mouse spleen is removed and placed in pre-cooled PBS containing 1% HSA. The spleen is cut with ophthalmic scissors, and the spleen tissue suspension is repeatedly blown and sucked with a pipette, then filtering through a 40 µm cell mesh, and centrifuging at 400 g for 5 min at room temperature. After centrifugation, the supernatant is discarded, and the spleen cells are used for later use.

1 ml of IX erythrocyte lysate is added to the spare bone marrow cells and spleen cells, vortexing to mix well, and lysing at room temperature for 15 min, during this period the sample centrifuge tube is inverted every 3 min. After the lysis, 4 ml of PBS containing 1% HSA is added to each sample to centrifuge at room temperature for 5 min at 400 g. After centrifugation, the supernatant is discarded, a and 1 ml of PBS containing 1% HSA is added to each sample to mix well by vortexing. 100 µl of cell suspension is taken out from each sample, then respectively adding antibodies according to the groups in Table 13, vortexing to mix well, and incubating at room temperature for 15 min in the dark. After the incubation, 5 µl of 7-AAD dye is added to each experimental sample, vortexing to mix well, and incubating at room temperature for 5 min in the dark. After the incubation, 1 ml of PBS containing 1% HSA is added to the NC and each sample to mix well, then centrifuging at 400 g for 5 min at room temperature. After centrifugation, the supernatant is discarded, and 100 µl of PBS containing 1% HSA is added to each experimental sample to resuspend the cells. The samples are stored at room temperature in the dark before testing, and detected by flow cytometry.

The test results are analyzed as follows: 1) the target cell population is human CD45+ cell population, human CD19+ cell population, human CD3+ cell population, human CD33+ cell population, and human CD56+ cell population; 2) the determination of the logic gate and gate position is shown in the Fig. 20: firstly the cell population is delineated as P1 gate; the adherent cells are removed in the cell population derived from the P1 gate as the P2 gate; the viable cell population in the cell population derived from P2 gate is delineated with 7-AAD as P3 gate; mouse CD45+ (P4 gate) and human CD45+ cell populations (P5 gate) in cell population derived from P3 gate are delineated with NC; human CD33+ cell population (P6 gate) and human CD56+ cell population (P7 gate) in cell population derived from P5 gate are delineated with NC; human CD19+ cell population (P8 gate) and human CD3+ cell population (P9 gate) in cell population derived from P5 gate are delineated with NC. The transplantation efficiency of human hematopoietic stem cells is expressed by the proportion of human CD45 cells, and the calculation method is as follows: human CD45% / (human CD45%+mouse CD45%). The efficiency of human hematopoietic stem cells differentiated into blood cells of various lineages in mice is expressed by human CD19% (representing B cells), human CD3% (representing T cells), human CD33% (representing myeloid cells), and human CD56% (representing NK cells).

The results in Fig. 21 show that, the transplantation efficiency of Dasatinib-treated cells is significantly higher than that of the Mock group and the SRI group in the peripheral blood detection at week 8, week 12, and week 16 under the condition of the same amount of cells in the initial culture for mouse transplantation. In bone marrow and spleen assays at week 16, the transplantation efficiency of the Dasatinib-treated cell group is significantly higher than that in the SRI group. It is proved that Dasatinib can improve the transplantation ability of hematopoietic stem cells, and the effect is better than that of the small molecule SRI reported in the literature.

The results in Fig. 22 show that, human-derived T cells, B cells, myeloid cells and NK cells can be detected in the peripheral blood, bone marrow, and spleen of mice at 16 weeks after transplantation, and the proportions between the cells of each lineage in each group are not significantly different. It is proved that human-derived hematopoietic stem cells are not only successfully transplanted, but can also differentiate into cells of various lineages with normal differentiation function.

## Claims

1. A method for promoting the proliferation of HSCs and maintaining the sternness of the HSCs, comprising in vitro contacting the HSCs with a culture medium containing a small molecule inhibitor of the STAT cell signaling pathway or a small molecule inhibitor of other cell signaling pathway than the STAT cell signaling pathway.

2. The method according to claim 1, wherein the small molecule inhibitor of the STAT cell signaling pathway is a small molecule inhibitor targeting Src.

3. The method according to claim 2, wherein the small molecule inhibitor targeting Src is one or more selected from the group consisting of: Dasatinib, Quercetin, UM-164, KX2-391, and KX1-004.

4. The method according to any one of claims 2-3, wherein the small molecule inhibitor targeting Src is used in combination with the small molecule inhibitor of the other cell signaling pathway.

5. The method according to claim 4, wherein the small molecule inhibitor of the other cell signaling pathway is one or two or more selected from the group consisting of: a small molecule inhibitor targeting HDAC, a small molecule inhibitor targeting PKC, and a small molecule inhibitor targeting JNK.

6. The method according to claim 5, wherein the small molecule inhibitor targeting Src is used in combination with a small molecule inhibitor targeting HDAC, a small molecule inhibitor targeting PKC, or a small molecule inhibitor targeting JNK.

7. The method according to claim 6, wherein the small molecule inhibitor targeting Src is used in combination with the small molecule inhibitor VPA targeting HDAC, the small molecule inhibitor SAHA targeting HDAC, the small molecule inhibitor Enzastaurin targeting PKC, or the small molecule inhibitor JNK-IN-8 targeting JNK.

8. The method according to claim 7, wherein the small molecule inhibitor targeting Src is Dasatinib.

9. The method according to claim 8, wherein Dasatinib is used in combination with VPA or SAHA.

10. The method according to any one of claims 2-8, wherein the small molecule inhibitor targeting Src is used alone or in combination with other inhibitor(s) to maintain the HSCs with a CD34+CD45+CD90+CD45RA-CD38- phenotype accounting for more than 8%, 10%, 15%, 20%, 25%, or 30% of all cells.

11. The method according to any one of claims 2-9, wherein the small molecule inhibitor targeting Src is used alone or in combination with other inhibitor(s) to maintain CD34+ cells accounting for more than 65%, 70%, 75%, 80%, or 85% of all cells.

12. A composition for maintaining the sternness of HSCs, comprising a small molecule inhibitor of the STAT cell signaling pathway.

13. The composition according to claim 12, wherein the small molecule inhibitor of the STAT cell signaling pathway is a small molecule inhibitor targeting Src.

14. The composition according to claim 13, wherein the small molecule inhibitor targeting Src is one or more selected from the group consisting of: Dasatinib, Quercetin, UM-164, KX2-391, and KX1-004.

15. The composition according to any one of claims 13-14, further comprising a small molecule inhibitor of other cell signaling pathway than the STAT cell signaling pathway.

16. The composition according to claim 15, wherein the small molecule inhibitor of theother cell signaling pathways comprises a small molecule inhibitor targeting HDAC, a small molecule inhibitor targeting PKC, or a small molecule inhibitor targeting JNK.

17. The composition according to claim 16, wherein the small molecule inhibitor of theother cell signaling pathways comprises a small molecule inhibitor VPA targeting HDAC, a small molecule inhibitor SAHA targeting HDAC, a small molecule inhibitor Enzastaurin targeting PKC, or a small molecule inhibitor JNK-IN-8 targeting JNK.

18. The composition according to any one of claims 12-17, further comprising SFEMII medium, growth factor Flt-3L, growth factor SCF, growth factor TPO, or growth factor IL-6.

19. The composition according to any one of claims 12-18, wherein the composition maintains HSCs with a CD34+CD45+CD90+CD45RA-CD38- phenotype accounting for more than 8%, 10%, 15%, 20%, 25%, or 30% of all cells.

20. The composition according to any one of claims 12-19, wherein the composition maintains CD34+ cells accounting for more than 65%, 70%, 75%, 80% or 85% of all cells.

21. A composition for maintaining the sternness of HSCs, comprising any combination selected from: SAHA+EPZ004777, SAHA+DZNeP, SAHA+Dasatinib, VPA+Dasatinib, SAHA+JNK-IN-8, or SAHA+VPA.

22. The composition according to claim 21, wherein the composition maintains the HSCs with CD34+CD45+CD90+CD45RA-CD38- phenotype accounting for more than 8%, 10%, 15%, 20%, 25% %, or 30% of all cells.

23. The composition according to claim 21 or 22, wherein the composition maintains CD34+ cells accounting for more than 65%, 70%, 75%, 80%, or 85% of all cells.

24. The composition according to any one of claims 21-23, further comprising SFEMII medium, growth factor Flt-3L, growth factor SCF, growth factor TPO and growth factor IL-6.

25. A composition for maintaining the sternness of HSCs, comprising any combination selected from: SAHA+EPZ004777+DZNeP, or SAHA+VPA+Dasatinib.

26. The composition according to claim 25, wherein the composition maintains the HSCs with CD34+CD45+CD90+CD45RA-CD38- phenotype accounting for more than 8%, 10%, 15%, 20%, 25%, or 30% of all cells.

27. The composition according to claim 25 or 26, wherein the composition maintains CD34+ cells accounting for more than 65%, 70%, 75%, 80%, or 85% of all cells.

28. The composition according to any one of claims 25-27, further comprising SFEMII medium, growth factor Flt-3L, growth factor SCF, growth factor TPO and growth factor IL-6.

29. The composition according to any one of claims 17-19, wherein the concentration of each inhibitor in the culture medium is:
Dasatinib: 0.1µM-50µM, preferably 0.5µM-40µM, more preferably 0.5µM-30µM, most preferably 0.5µM-10µM;
SAHA: 10nM-20µM, preferably 20nM-15µM, more preferably 30nM-10µM, most preferably 0.1µm-10µM;
VPA: 10µM-2000µM, preferably 10µM-1500µM, more preferably 10µM-1000µM, most preferably 100µM-1000µM;
JNK-IN-8: 0.1 µM-20 µM, preferably 0.5 µM-15 µM, more preferably 0.5 µM-10 µM, most preferably 1 µM-10 µM;
EPZ004777: 0.1 µM-50 µM, preferably 0.5 µM-40 µM, more preferably 0.5 µM-30 µM, most preferably 0.5 µM-10 µM;
DZNeP: 1 nM-500 nM, preferably 5 nM-400 nM, more preferably 10 nM-300 nM, most preferably 10 nM-250 nM;
UM-164: 0.1 µM-1000 µM, preferably 0.5 µM-500 µM, more preferably 1 µM-100 µM, most preferably 1 µM-10 µM;
KX2-391: 0.1 nM-1000 nM, preferably 1 nM-1000 nM, more preferably 10 nM-500 nM, most preferably 10 nM-100 nM;
KX1-004: 0.1 µM-1000 µM, preferably 1 µM-1000 µM, more preferably 10 µM-500 µM, most preferably 10 µM-100 µM.

30. The composition according to any one of claims 21-24, wherein the concentration of each inhibitor in the culture medium is:
Dasatinib: 0.1 µM-50 µM, preferably 0.5 µM-40 µM, more preferably 0.5 µM-30 µM, most preferably 0.5 µM-10 µM;
SAHA: 10 nM-20 µM, preferably 20 nM-15 µM, more preferably 30 nM-10 µM, most preferably 0.1 µM-10 µM;
VPA: 10 µM-2000 µM, preferably 10 µM-1500 µM, more preferably 10 µM-1000 µM, most preferably 100 µM-1000 µM;
JNK-IN-8: 0.1 µM-20 µM, preferably 0.5 µM-15 µM, more preferably 0.5 µM-10 µM, most preferably 1 µM-10 µM;
EPZ004777: 0.1 µM-50 µM, preferably 0.5 µM-40 µM, more preferably 0.5 µM-30 µM, most preferably 0.5 µM-10 µM;
DZNeP: 1 nM-500 nM, preferably 5 nM-400 nM, more preferably 10 nM-300 nM, most preferably 10 nM-250 nM.

31. A composition according to any one of claims 25-28, wherein the concentration of each inhibitor in the culture medium is:
Dasatinib: 0.1 µM-50 µM, preferably 0.5 µM-40 µM, more preferably 0.5 µM-30 µM, most preferably 0.5 µM-10 µM;
SAHA: 10 nM-20 µM, preferably 20 nM-15 µM, more preferably 30 nM-10 µM, most preferably 0.1 µM-10 µM;
VPA: 10 µM-2000 µM, preferably 10 µM-1500 µM, more preferably 10 µM-1000 µM, most preferably 100 µM-1000 µM;
EPZ004777: 0.1 µM-50 µM, preferably 0.5 µM-40 µM, more preferably 0.5 µM-30 µM, most preferably 0.5 µM-10 µM;
DZNeP: 1 nM-500 nM, preferably 5 nM-400 nM, more preferably 10 nM-300 nM, most preferably 10 nM-250 nM.
